# EUROPEAN PATENT APPLICATION

(11) **EP 4 379 738 A1**
(43) Date of publication of application: **05.06.2024**
(21) Application number: 22801687.9
(22) Date of filing: 29.07.2022
(51) Int. Cl.: G16H 50/50, G16H 50/70, G16H 50/20, G06N 20/00, A61B 5/00, A61B 5/024

(54) **THYROID DYSFUNCTION PREDICTION METHOD AND SYSTEM FOR SUBJECT**

(30) Priority: 29.07.2021 KR 20210099606
(71) Applicant: Thyroscope Inc., Ulju-gun, Ulsan 44919 (KR)
(72) Inventor: SHIN, Kyubo, Ulju-gun Ulsan 44951 (KR); MOON, Jae Hoon, Seoul 06189 (KR); KIM, Jongchan, Ulju-gun Ulsan 44969 (KR); PARK, Jaemin, Ulju-gun Ulsan 44930 (KR); LEE, Juhui, Ulsan 44236 (KR)
(74) Representative: Dantz, Jan Henning
(86) International application number: PCT/KR2022/011163
(87) International publication number: WO 2023/008938

(57) **Abstract**

According to the present application, a method for predicting thyroid dysfunction for a subject is disclosed. The method comprises, determining a target data based on a trigger signal; obtaining interval heart rates corresponding to the determined target date; obtaining, for the subject, a first pre-processing result for the obtained interval heart rates corresponding to the determined target date, wherein the first pre-processing result includes at least one parameter related to an average and distribution of the interval heart rates corresponding to the target date; obtaining, for the subject, at least one of concentration of hormone related to a thyroid corresponding to a reference date; obtaining, for the subject, a second pre-processing result for interval heart rates corresponding to the reference date, wherein the second pre-processing result includes at least one parameter related to an average and distribution of the interval heart rates corresponding to the reference date; obtaining a difference of the first pre-processing result with respect to the second pre-processing result; and obtaining a prediction result for thyroid dysfunction obtained based on values including the at least one of concentration of hormone related to a thyroid corresponding to a reference date and the difference.

## Description

### Technical Field

The present application relates to a method for analyzing a patient's heart rate and predicting thyroid dysfunction on the basis of a result of the analysis, and to an apparatus and a system for performing the method.

### Background Art

The thyroid is an endocrine organ at the front of the neck, and produces thyroid hormone by receiving signals of thyroid stimulating hormone secreted by the pituitary gland in the brain.

The thyroid hormone produced by the thyroid regulates the metabolic rate of a person's body. When the thyroid hormone is secreted more than a normal level, the body metabolism progresses abnormally rapidly, and symptoms, such as feeling hotter than usual or feeling the heart pounding, appear. Conversely, when the thyroid hormone is secreted less than the normal level, symptoms, such as feeling very cold, feeling tired and depressed, or having a slow pulse, appear.

When the function of the thyroid is normal, the secretion of the thyroid hormone occurs without any problem. However, when the function of the thyroid is abnormal, the secretion of the thyroid hormone is decreased or increased. A series of diseases caused by the decrease of the secretion of the thyroid hormone are called hypothyroidism, and a series of diseases caused by the increase of the secretion of the thyroid hormone are called hyperthyroidism. Furthermore, these two diseases area called thyroid dysfunction.

Currently, the only diagnostic method for checking thyroid dysfunction is to check the concentration of the thyroid hormone in a patient's blood and to determine whether the concentration of the thyroid hormone is within a normal range, at a high level, or a low level, in terms of clinical pathology.

In the meantime, there are several methods to treat hyperthyroidism and hypothyroidism, but the most widely used treatment method is a drug treatment method. Most patients suffering from hyperthyroidism and most patients suffering from hypothyroidism recover normal levels of thyroid hormone generation and secretion functions by taking drugs for a predetermined period of time. However, hyperthyroidism and hypothyroidism are diseases that have a low cure rate and a very high recurrence rate, so it is necessary to continuously monitor whether hyperthyroidism or hypothyroidism recurs.

However, as described above, since the only diagnostic method for checking thyroid functions is to check the concentration of the hormone through blood collecting, there is a problem in that a patient should visit a hospital periodically in order to monitor whether the thyroid is dysfunctional.

Accordingly, there is a demand for the development of a technology for checking whether the thyroid is dysfunctional, more accurately by a method other than checking a hormone level through blood collecting.

That is, it is desired to develop a method of enabling individual patients to monitor thyroid dysfunction more simply and quickly without visiting a hospital in person, and of inducing a patient to visit a hospital when necessary.

### Disclosure

### Technical Problem

The problem to be solved by contents disclosed by the present application is to providing a prediction model capable of predicting a thyroid dysfunction, on the basis of heart rate information obtained using a personal electronic device that ordinary people can use rather than a professional medical diagnostic device.

Another problem to be solved by contents disclosed by the present application is to providing a training method of the above-described prediction model capable of predicting a thyroid dysfunction, on the basis of heart rate information.

Another problem to be solved by contents disclosed by the present application is d to providing a method and a system for enabling ordinary people to continuously monitor thyroid dysfunction by using the above-described prediction model capable of predicting a thyroid dysfunction, on the basis of heart rate information without a doctor's help and a hospital visit in person.

Technical problems to be solved by the present application are not limited to the aforementioned technical problems and other technical problems which are not mentioned will be clearly understood by those skilled in the art from the present specification and the accompanying drawings.

### Technical Solution

According to an embodiment of the present application, a method for predicting thyroid dysfunction for a subject is disclosed. A method for predicting thyroid dysfunction for a subject, comprising: obtaining a trigger signal; determining a target date based on the obtained trigger signal; obtaining interval heart rates corresponding to the determined target date; obtaining, for the subject, a first pre-processing result for the obtained interval heart rates corresponding to the determined target date, wherein the first pre-processing result includes at least one parameter related to an average and distribution of the interval heart rates corresponding to the target date; obtaining, for the subject, at least one of concentration of hormone related to a thyroid corresponding to a reference date; obtaining, for the subject, a second pre-processing result for interval heart rates corresponding to the reference date, wherein the second pre-processing result includes at least one parameter related to an average and distribution of the interval heart rates corresponding to the reference date; obtaining a difference of the first pre-processing result with respect to the second pre-processing result; and obtaining a prediction result for thyroid dysfunction obtained based on values including the at least one of concentration of hormone related to a thyroid corresponding to a reference date and the difference; wherein the prediction result for thyroid dysfunction is a result of processing input values by a thyroid dysfunction prediction model, wherein the input values include the difference and the at least one of concentration of hormone related to a thyroid corresponding to the reference date.

In some embodiments disclosed by the present application, the at least one parameter related to the distribution of the interval heart rates corresponding to the target date includes standard deviation, skewness and kurtosis of the interval heart rates corresponding to the target date, wherein the at least one parameter related to the distribution of the interval heart rates corresponding to the reference date includes standard deviation, skewness and kurtosis of the interval heart rates corresponding to the reference date.

In some embodiments disclosed by the present application, the difference of the first pre-processing result with respect to the second pre-processing result is one selected from a group consisting of 1) an amount of change in the average of the interval heart rates of the target date with respect to the average of the interval heart rates of the reference date, 2) a rate of change in the average of the interval heart rates of the target date with respect to the average of the interval heart rates of the reference date, 3) an amount of change in a standard deviation of the interval heart rates of the target date with respect to a standard deviation of the interval heart rates of the reference date, 4) an amount of change in a relative standard deviation of the interval heart rates of the target date with respect to a relative standard deviation of the interval heart rates of the reference date, 5) an amount of change in a skewness of the interval heart rates of the target date with respect to a skewness of the interval heart rates of the reference date, 6) an amount of change in a kurtosis of the interval heart rates of the target date with respect to a kurtosis of the interval heart rates of the reference date, 7) a JS Divergence between the interval heart rates corresponding to the reference date and the interval heart rates corresponding to the target date or a combination thereof.

In some embodiments disclosed by the present application, the at least one of concentration of hormone corresponding to the reference date is one selected from a group consisting of 1) concentration of thyroid stimulating hormone(TSH), 2) concentration of tetraiodothyronine(T4), 3) concentration of free T4(free T4) in serum, 4) concentration of triiodothyronine(T3), 5) concentration of free T3(free T3) in serum, 6) concentration of thyrotropin-releasing hormone(TRH) or a combination thereof.

In some embodiments disclosed by the present application, the method further comprises : obtaining a first value and a second value from a group consisting of 1) an amount of change in the average of the interval heart rates of the target date with respect to the average of the interval heart rates of the reference date, 2) a rate of change in the average of the interval heart rates of the target date with respect to the average of the interval heart rates of the reference date, 3) an amount of change in a standard deviation of the interval heart rates of the target date with respect to a standard deviation of the interval heart rates of the reference date, 4) an amount of change in a relative standard deviation of the interval heart rates of the target date with respect to a relative standard deviation of the interval heart rates of the reference date, 5) an amount of change in a skewness of the interval heart rates of the target date with respect to a skewness of the interval heart rates of the reference date, 6) an amount of change in a kurtosis of the interval heart rates of the target date with respect to a kurtosis of the interval heart rates of the reference date, 7) a JS Divergence between the interval heart rates corresponding to the reference date and the interval heart rates corresponding to the target date 8) concentration of thyroid stimulating hormone(TSH) of the subject corresponding to the reference date, 9) concentration of tetraiodothyronine(T4) of the subject corresponding to the reference date, 10) concentration of free T4(free T4) in the subject's serum corresponding to the reference date, 11) concentration of triiodothyronine(T3) of the subject corresponding to the reference date, 12) concentration of free T3(free T3) in the subject's serum corresponding to the reference date, 6) concentration of thyrotropin-releasing hormone(TRH) of the subject corresponding to the reference date, obtaining any one value among i) a value obtained by multiplying the first value by the second value, ii) a value obtained by dividing the first value by the second value and iii) a value obtained by dividing the second value by the first value.

In some embodiments disclosed by the present application, the obtaining the prediction result for thyroid dysfunction based on values including the at least one of concentration of hormone corresponding to the reference date and the difference is obtaining the prediction result for thyroid dysfunction based on values including at least one of concentration of hormone corresponding to the reference date, the difference and the obtained any one value, wherein the prediction result for thyroid dysfunction is a result of processing input values by the thyroid dysfunction prediction model, wherein the input values include the difference, the obtained any one value and at least one of concentration of hormone corresponding to the reference date.

In some embodiments disclosed by the present application, the method further comprises : obtaining a day gap between the target date and the reference date.

Herein, the obtaining the prediction result for thyroid dysfunction based on values including the at least one of concentration of hormone corresponding to the reference date and the difference is obtaining the prediction result for thyroid dysfunction based on values including at least one of concentration of hormone corresponding to the reference date, the difference and the day gap between the target date and the reference date, wherein the prediction result for thyroid dysfunction is a result of processing input values by the thyroid dysfunction prediction model, wherein the input values include the difference, the day gap between the target date and the reference date and at least one of concentration of hormone corresponding to the reference date.

In some embodiments disclosed by the present application, the interval heart rates corresponding to the target date are all resting heart rates corresponding to a predetermined period based on the target date, wherein the interval heart rates corresponding to the reference date are all resting heart rates corresponding to the predetermined period based on the reference date.

Herein, the predetermined period is any one selected from 8,9,10,11,12,13,14,15,16 and 17 days.

In some embodiments disclosed by the present application, the thyroid dysfunction prediction model includes a hyperthyroidism prediction model and a hypothyroidism prediction model.

Herein, the prediction result for thyroid dysfunction is determined by considering a first prediction result in which the input values including at least one hormone concentration and the difference are processed by the hyperthyroidism prediction model and a second prediction result in which the input values including at least one hormone concentration and the difference are processed by the hypothyroidism prediction model.

### Advantageous Effects

According to the disclosure in the present application, a prediction model capable of predicting a thyroid dysfunction, on the basis of heart rate information obtained using a personal electronic device that ordinary people can use rather than a professional medical diagnostic device can be provided.

According to the disclosure in the present application, a training method of the above-described prediction model capable of predicting a thyroid dysfunction, on the basis of heart rate information can be provided.

According to the disclosure in the present application, a method and a system for enabling ordinary people to continuously monitor thyroid dysfunction by using the above-described prediction model capable of predicting a thyroid dysfunction, on the basis of heart rate information without a doctor's help and a hospital visit in person can be provided.

### Description of Drawings

FIG. 1 is a diagram illustrating a system for predicting thyroid dysfunction according to an embodiment described in the present application.
FIG. 2 is a block diagram illustrating a heart rate measuring device described in the present application.
FIG. 3 is a block diagram illustrating a user terminal described in the present application.
FIG. 4 is a block diagram illustrating a server described in the present application.
FIG. 5 is a flowchart illustrating a method for predicting thyroid dysfunction described in the present application.
FIG. 6 is a flowchart illustrating a method for predicting thyroid dysfunction described in the present application.

### Mode for Invention

The above-described objectives, features, and advantages of the present application will be more apparent from the following detailed description with reference to the accompanying drawings. In addition, various modifications may be made to the present application, and various embodiments of the present application may be practiced. Therefore, specific embodiments will be described in detail below with reference to the accompanying drawings.

Throughout the specification, the same reference numerals denote the same elements in principle. In addition, elements having the same function within the same scope illustrated in the drawings of the embodiments are described using the same reference numerals, and a redundant description will be omitted.

A detailed description of a well-known function or configuration relating to the present application is omitted when determined as obfuscating the nature and gist of the present application. In addition, throughout the present specification, the terms first, second, and so on are used only to distinguish from one element to another.

In addition, the terms "module" and "part" that are used to name an element in the description below are used considering only the ease with which the present specification is written. The terms are not intended as having different special meanings or functions and thus may be used individually or interchangeably.

In the following embodiments, an expression used in the singular encompasses the expression of the plural, unless it has a clearly different meaning in the context.

In the following embodiments, it is to be understood that terms such as "including", "having", etc. are intended to indicate the existence of features or elements disclosed in the specification, and are not intended to preclude the possibility that one or more other features or elements may be added.

Sizes of elements in the drawings may be exaggerated or reduced for convenience of description. For example, any size and thickness of each element shown in the drawings are shown for convenience of description, and the present disclosure is not limited thereto.

In a case in which a particular embodiment is realized otherwise, a particular process may be performed out of the order described. For example, two processes described in succession may be performed substantially simultaneously, or may proceed in the order opposite to the order described.

In the following embodiments, when elements are referred to as being connected to each other, the elements are directly connected to each other or the elements are indirectly connected to each other with intervening elements therebetween. For example, in the present specification, when elements are referred to as being electrically connected to each other, the elements are directly electrically connected to each other or the elements are indirectly electrically connected with intervening elements therebetween.

According to an aspect of the present application, a method for predicting thyroid dysfunction for a subject is disclosed. The method for predicting thyroid dysfunction for a subject is comprising: obtaining a trigger signal; determining a target date based on the obtained trigger signal; obtaining interval heart rates corresponding to the determined target date; obtaining, for the subject, a first pre-processing result for the obtained interval heart rates corresponding to the determined target date, wherein the first pre-processing result includes at least one parameter related to an average and distribution of the interval heart rates corresponding to the target date; obtaining, for the subject, at least one of concentration of hormone related to a thyroid corresponding to a reference date; obtaining, for the subject, a second pre-processing result for interval heart rates corresponding to the reference date, wherein the second pre-processing result includes at least one parameter related to an average and distribution of the interval heart rates corresponding to the reference date; obtaining a difference of the first pre-processing result with respect to the second pre-processing result; and obtaining a prediction result for thyroid dysfunction obtained based on values including the at least one of concentration of hormone related to a thyroid corresponding to a reference date and the difference; wherein the prediction result for thyroid dysfunction is a result of processing input values by a thyroid dysfunction prediction model, wherein the input values include the difference and the at least one of concentration of hormone related to a thyroid corresponding to the reference date.

In some embodiments disclosed by the present application, wherein the at least one parameter related to the distribution of the interval heart rates corresponding to the target date includes standard deviation, skewness and kurtosis of the interval heart rates corresponding to the target date, wherein the at least one parameter related to the distribution of the interval heart rates corresponding to the reference date includes standard deviation, skewness and kurtosis of the interval heart rates corresponding to the reference date.

In some embodiments disclosed by the present application, wherein the difference of the first pre-processing result with respect to the second pre-processing result is one selected from a group consisting of 1) an amount of change in the average of the interval heart rates of the target date with respect to the average of the interval heart rates of the reference date, 2) a rate of change in the average of the interval heart rates of the target date with respect to the average of the interval heart rates of the reference date, 3) an amount of change in a standard deviation of the interval heart rates of the target date with respect to a standard deviation of the interval heart rates of the reference date, 4) an amount of change in a relative standard deviation of the interval heart rates of the target date with respect to a relative standard deviation of the interval heart rates of the reference date, 5) an amount of change in a skewness of the interval heart rates of the target date with respect to a skewness of the interval heart rates of the reference date, 6) an amount of change in a kurtosis of the interval heart rates of the target date with respect to a kurtosis of the interval heart rates of the reference date, 7) a JS Divergence between the interval heart rates corresponding to the reference date and the interval heart rates corresponding to the target date or a combination thereof.

In some embodiments disclosed by the present application, wherein the at least one of concentration of hormone corresponding to the reference date is one selected from a group consisting of 1) concentration of thyroid stimulating hormone(TSH), 2) concentration of tetraiodothyronine(T4), 3) concentration of free T4(free T4) in serum, 4) concentration of triiodothyronine(T3), 5) concentration of free T3(free T3) in serum, 6) concentration of thyrotropin-releasing hormone(TRH) or a combination thereof.

In some embodiments disclosed by the present application, wherein the method further comprises : obtaining a first value and a second value from a group consisting of 1) an amount of change in the average of the interval heart rates of the target date with respect to the average of the interval heart rates of the reference date, 2) a rate of change in the average of the interval heart rates of the target date with respect to the average of the interval heart rates of the reference date, 3) an amount of change in a standard deviation of the interval heart rates of the target date with respect to a standard deviation of the interval heart rates of the reference date, 4) an amount of change in a relative standard deviation of the interval heart rates of the target date with respect to a relative standard deviation of the interval heart rates of the reference date, 5) an amount of change in a skewness of the interval heart rates of the target date with respect to a skewness of the interval heart rates of the reference date, 6) an amount of change in a kurtosis of the interval heart rates of the target date with respect to a kurtosis of the interval heart rates of the reference date, 7) a JS Divergence between the interval heart rates corresponding to the reference date and the interval heart rates corresponding to the target date 8) concentration of thyroid stimulating hormone(TSH) of the subject corresponding to the reference date, 9) concentration of tetraiodothyronine(T4) of the subject corresponding to the reference date, 10) concentration of free T4(free T4) in the subject's serum corresponding to the reference date, 11) concentration of triiodothyronine(T3) of the subject corresponding to the reference date, 12) concentration of free T3(free T3) in the subject's serum corresponding to the reference date, 13) concentration of thyrotropin-releasing hormone(TRH) of the subject corresponding to the reference date, obtaining any one value among i) a value obtained by multiplying the first value by the second value, ii) a value obtained by dividing the first value by the second value and iii) a value obtained by dividing the second value by the first value

Herein, the obtaining the prediction result for thyroid dysfunction based on values including the at least one of concentration of hormone corresponding to the reference date and the difference is obtaining the prediction result for thyroid dysfunction based on values including at least one of concentration of hormone corresponding to the reference date, the difference and the obtained any one value, wherein the prediction result for thyroid dysfunction is a result of processing input values by the thyroid dysfunction prediction model, wherein the input values include the difference, the obtained any one value and at least one of concentration of hormone corresponding to the reference date.

In some embodiments disclosed by the present application, wherein the method further comprises : obtaining a day gap between the target date and the reference date.

Herein, the obtaining the prediction result for thyroid dysfunction based on values including the at least one of concentration of hormone corresponding to the reference date and the difference is obtaining the prediction result for thyroid dysfunction based on values including at least one of concentration of hormone corresponding to the reference date, the difference and the day gap between the target date and the reference date, wherein the prediction result for thyroid dysfunction is a result of processing input values by the thyroid dysfunction prediction model, wherein the input values include the difference, the day gap between the target date and the reference date and at least one of concentration of hormone corresponding to the reference date.

In some embodiments disclosed by the present application, wherein the interval heart rates corresponding to the target date are all resting heart rates corresponding to a predetermined period based on the target date, wherein the interval heart rates corresponding to the reference date are all resting heart rates corresponding to the predetermined period based on the reference date.

Herein, the predetermined period is any one selected from 8,9,10,11,12,13,14,15,16 and 17 days.

In some embodiments disclosed by the present application, wherein the thyroid dysfunction prediction model includes a hyperthyroidism prediction model and a hypothyroidism prediction model.

Herein, the prediction result for thyroid dysfunction is determined by considering a first prediction result in which the input values including at least one hormone concentration and the difference are processed by the hyperthyroidism prediction model and a second prediction result in which the input values including at least one hormone concentration and the difference are processed by the hypothyroidism prediction model.

### 1. Definitions of Terms

### Heart Rate

In the present specification, the term "heart rate" refers to the number of heartbeats per unit time. For example, heart rate may be measured as beats per minute (bpm). In the meantime, in the present specification, unless otherwise specified or the term is used for the purpose of general scientific explanation, "heart rate" is construed as corresponding to or associated with the time point (estimation time point) at which the heart rate is measured.

### Resting Period

In the present specification, the term "resting period" refers to a period during which a person's physical movement is at or below a particular level. In general, a "resting period" refers to a period during which a subject is awake and motionless, but the term "resting period" in the present specification also refers to a period during which a subject is asleep.

### Resting Heart Rates

In the present specification, the term "resting heart rates" refer to heart rates for which the time points at which the heart rates are measured are within a person's resting period.

In particular, the expression "resting heart rates corresponding to day X" in the present specification may refer to all heart rates corresponding to a resting period included in the period from 12:00:00 a.m. on day X to 11:59:59 p.m. on day X.

However, considering that i) a resting period generally includes a person's sleeping hours, and that ii) many people start to sleep before 12:00:00 a.m. taking people's sleeping habits into consideration, "resting heart rates corresponding to day X" in the present specification may include resting heart rates corresponding to the time period from the time at which a person starts to sleep on the day immediately before day X (hereinafter, referred to as day X-1) to 11:59:59 p.m. on day X-1. Similarly, "resting heart rates corresponding to day X" may not include resting heart rates corresponding to the time period from the time at which a person starts to sleep on day X to 11 :59:59 p.m. on day X. That is, resting heart rates corresponding to the time period from the time at which a person starts to sleep on day X to 11 :59:59 p.m. on day X may be included in "resting heart rates corresponding to the day (hereinafter, referred to as day X+1) after day X" as described above.

### Interval Heart Rates (Period Heart Rates)

In the present specification, the term "interval heart rates (period heart rates)" refer to all resting heart rates corresponding to a period of two days or more. In the present specification, the term "interval resting heart rates (period resting heart rates)" and the term "interval heart rates" may be used as having the same meaning.

### Interval average heart rate (Period Average Heart Rate)

In the present specification, the term "interval average heart rate (period average heart rate)" refers to an average value of resting heart rates measured for a period of two days or more (that is, interval heart rates). For example, a period average heart rate for five days refers to an average value of a resting heart rates corresponding to a first day, a resting heart rates corresponding to a second day, a resting heart rates corresponding to a third day, a resting heart rates corresponding to a fourth day, a the resting heart rates corresponding to a fifth day. In the present specification, the term "average of interval heart rates (average of period heart rates)" and the term "period average heart rate" may be used as having the same meaning.

### Thyroid Hormone

In the present specification, the term "thyroid hormone" has meanings including: thyroxine (T4) or tetraiodothyronine, which are hormones secreted by the thyroid; serum free T4, which is free T4 in serum; triiodothyronine (T3) resulting from conversion to remove iodine from T4; serum free T3, which is free T3 in serum; thyroid stimulating hormone (TSH) secreted by the pituitary gland; and thyrotropin-releasing hormone (TRH).

In the meantime, in the present specification, the term "hormone" and the term "thyroid hormone" may be used as having the same meaning.

### Hyperthyroidism

In the present specification, the term "hyperthyroidism" refers to a condition in which thyroid hormone concentration in blood is excessively increased because hormones secreted by the thyroid are excessively produced or secreted or because of other factors, and to all clinical pathological symptoms resulting therefrom.

### Hypothyroidism

In the present specification, the term "hypothyroidism" refers to a condition in which thyroid hormone concentration in blood is excessively decreased because hormones secreted by the thyroid are too little produced or secreted or because of other factors, and to all clinical pathological symptoms resulting therefrom.

### Thyroid Dysfunction

In the present specification, the term "thyroid dysfunction" has meanings including both "hypothyroidism" and "hyperthyroidism" described above.

### 2. Training Method

### Summary

In 2017, to solve the problem in the related art described above and to develop a technology that meets the needs of the market, the applicants of the present application completed the development of a technology for a method and a system, and applied for a patent related thereto. The method and the system is for determining whether a patient's current state is a state in which a thyroid function is normal or the current state is a state in which a thyroid function is abnormal (that is, hyperthyroidism or hypothyroidism), by using a heart rate corresponding to the time point at which it is determined a patient's thyroid function is "normal", and a current heart rate as main input variables, on the basis of a correlation between a heart rate and hyperthyroidism.

However, there were several problems in the technology that the applicants of the present application developed.

First, heart rates with a normal thyroid function (period average heart rates for a predetermined period) vary from person to person, so there was a difficulty in deriving a function of outputting whether the thyroid is dysfunctional, by simply using a heart rate in a normal state and a heart rate in a current state as factors.

Second, since a heart rate in a "normal" state was used as a factor, the above-described technology could not be applied when rather than "normal", "hyperthyroidism" or "hypothyroidism" was determined on the day of a hormone level test.

Because of these problems described above, the conventionally developed technology had low accuracy and was inconvenient in use, and was thus insufficient to meet the needs of the market that requires the necessity for continuous monitoring.

To solve these problems, the present applicants propose a machine learning-based thyroid dysfunction prediction model described in the present specification.

In addition, in training a prediction model for predicting a thyroid dysfunction, there were several problems in creating a personalized prediction model, so the present applicants analyzed attributes of data for training the prediction model, and trained the model in various ways with the purpose of increasing the accuracy of prediction, and thus found some meaningful training methods for increasing the accuracy of the prediction model, and discloses training methods for increasing accuracy in the present specification.

### Collection of Clinical Data

First described will be a method of collecting basic clinical data for training the prediction model described in the present application.
(1) Patients suffering from hyperthyroidism or hypothyroidism are selected.
(2) Personal information of the selected patients is obtained. For example, patients' personal information including names, sexes, ages, weights, heights, etc. of the selected patients is obtained.
(3) Each of the selected patients is given a wearable device that is wearable on his or her wrist and has a function of measuring the patient's heart rates.
(4) For each of the selected patients, when selected patients visit a hospital and have a thyroid hormone test, interval heart rates (period heart rates) corresponding to the test date are obtained among heart rates measured by the wearable devices provided to the patients.
(5) For each of the selected patients, concentration values of thyroid hormones on the test date are obtained.
(6) For each of the selected patients, a doctor's diagnosis result for the patient based on the thyroid hormone test result is obtained. That is, a thyroid hormone test result, and information on which state among hyperthyroidism, hypothyroidism, and normal the patient is in are obtained.

Consequently, if one patient is tested for a thyroid hormone test once, one data set may be obtained including: i) personal information of the patient, ii) the test date, iii) interval heart rates corresponding to the test date, iv) concentrations of thyroid hormones according to the test conducted on the test date, and v) a doctor's diagnosis result for a thyroid dysfunction according to a result of the test conducted on the test date.

That is, if data sets according to an average of four hormone test results per patient are obtained from 100 patients, a total of 400 data sets are obtained and if data sets according to an average of three hormone test results per patient are obtained from 300 patients, a total of 900 data sets are obtained.

Patients suffering from a thyroid dysfunction are recommended to visit a hospital once every three months and have a blood-collecting test for a hormone level test. Accordingly, basically, to sufficiently obtain data sets in the above-described way, it is necessary to increase the number of patients targeted for collection of data sets or to lengthen the period during which data sets per patient are obtained, to several years or longer. That is, the current situation is that obtaining considerable clinical data related to thyroid dysfunction requires the investment of enormous expenses or the investment of considerable time.

### Training Method Embodiment #1. Difference Value from Heart Rates on Reference Date, and Hormone Value on Reference date

According to a first exemplary embodiment described in the present application, in order to train a learning model, a value obtained by subtracting, from a period average heart rate corresponding to a first test date, a period average heart rate corresponding to a second test date, and a hormone concentration of the second test date may be used as input values, and a diagnosis result corresponding to the first test date may be used as a labeling value.

Herein, as the learning model, classification models including light gradient boosting machine, support vector machine, random forest, extra trees, ada boost, extreme gradient boosting, CatBoost, etc. may be used.

The learning model includes a hyperthyroidism prediction model for predicting hyperthyroidism and a hypothyroidism prediction model for predicting hypothyroidism.

According to the first exemplary embodiment, when the hyperthyroidism prediction model is trained, all the obtained clinical data cannot be used, and only clinical data of patients diagnosed with hyperthyroidism at least once as a diagnosis result may be used as training data.

Hereinafter, for convenience of description, a patient who has been diagnosed with hyperthyroidism at least once as a diagnosis result may be referred to as a 'hyperthyroidism patient'.

In the meantime, in calculating a period average heart rate, resting heart rates for a predetermined period predetermined based on a particular date may be used, wherein the predetermined period may be one of the following: 1, 2, 3, 4, 5, 6, 7, 8, 9, 10, 11, 12, 13, 14, 15, 16, 17, 18, 19, 20, 21, 22, 23, 24, 25, 26, 27, 28, 29, and 30 days.

According to the first exemplary embodiment, in order to train the hyperthyroidism prediction model, clinical data of hyperthyroidism patients may be used to generate training data sets in the format such as [(a period average heart rate corresponding to a first test date - a period average heart rate corresponding to a second test date), a thyroid hormone concentration corresponding to the second test date, a diagnosis result (hyperthyroidism or non-hyperthyroidism) corresponding to the first test date].

For example, assuming that a total of three data sets were obtained from a first hyperthyroidism patient, that a diagnosis result for the first test was 'hyperthyroidism' with a hormone concentration of a first concentration, that a diagnosis result for the second test was 'normal' with a hormone concentration of a second concentration, and that a diagnosis result for the third test was 'normal' with a hormone concentration of a third concentration, training data sets that may be obtained from the clinical data of the first hyperthyroidism patient may be generated as follows.
[(a period average heart rate corresponding to a first test date - a period average heart rate corresponding to a second test date), a second concentration, hyperthyroidism],
[(the period average heart rate corresponding to the first test date - a period average heart rate corresponding to a third test date), a third concentration, hyperthyroidism],
[(the period average heart rate corresponding to the second test date - the period average heart rate corresponding to the first test date), a first concentration, non-hyperthyroidism],
[(the period average heart rate corresponding to the second test date - the period average heart rate corresponding to the third test date), the third concentration, non-hyperthyroidism],
[(the period average heart rate corresponding to the third test date - the period average heart rate corresponding to the first test date), the first concentration, non-hyperthyroidism], and
[(the period average heart rate corresponding to the third test date - the period average heart rate corresponding to the second test date), the second concentration, non-hyperthyroidism],

As another example, assuming that a total to four data sets were obtained from a second hyperthyroidism patient, that a diagnosis result for the first test was 'hyperthyroidism' with a hormone concentration of a first concentration, that a diagnosis result for the second test was 'normal' with a hormone concentration of a second concentration, that a diagnosis result for the third test was 'hyperthyroidism' with a hormone concentration of a third concentration, and that a diagnosis result for the fourth test was 'normal' with a hormone concentration of a fourth concentration, training data sets that may be obtained from the clinical data of the second hyperthyroidism patient may be generated as follows.
[(a period average heart rate corresponding to a first test date - a period average heart rate corresponding to a second test date), a second concentration, hyperthyroidism],
[(the period average heart rate corresponding to the first test date - a period average heart rate corresponding to a third test date), a third concentration, hyperthyroidism],
[(the period average heart rate corresponding to the first test date - a period average heart rate corresponding to a fourth test date), a fourth concentration, hyperthyroidism],
[(the period average heart rate corresponding to the second test date - the period average heart rate corresponding to the first test date), a first concentration, non-hyperthyroidism],
[(the period average heart rate corresponding to the second test date - the period average heart rate corresponding to the third test date), the third concentration, non-hyperthyroidism],
[(the period average heart rate corresponding to the second test date - the period average heart rate corresponding to the fourth test date), the fourth concentration, non-hyperthyroidism],
[(the period average heart rate corresponding to the third test date - the period average heart rate corresponding to the first test date), the first concentration, hyperthyroidism],
[(the period average heart rate corresponding to the third test date - the period average heart rate corresponding to the second test date), the second concentration, hyperthyroidism],
[(the period average heart rate corresponding to the third test date - the period average heart rate corresponding to the fourth test date), the fourth concentration, hyperthyroidism],
[(the period average heart rate corresponding to the fourth test date - the period average heart rate corresponding to the first test date), the first concentration, non-hyperthyroidism],
[(the period average heart rate corresponding to the fourth test date - the period average heart rate corresponding to the second test date), the second concentration, non-hyperthyroidism], and
[(the period average heart rate corresponding to the fourth test date - the period average heart rate corresponding to the third test date), the third concentration, non-hyperthyroidism],

According to the first exemplary embodiment, when the hypothyroidism prediction model is trained, all the obtained clinical data cannot be used, and only clinical data of patients diagnosed with hypothyroidism at least once as a diagnosis result may be used as training data.

Hereinafter, for convenience of description, a patient who has been diagnosed with hypothyroidism at least once as a diagnosis result may be referred to as a 'hypothyroidism patient'.

According to the first exemplary embodiment, in order to train the hypothyroidism prediction model, clinical data of hypothyroidism patients may be used to generate training data sets in the format such as [(a period average heart rate corresponding to a first test date - a period average heart rate corresponding to a second test date), a thyroid hormone concentration corresponding to the second test date, a diagnosis result (hypothyroidism or non-hypothyroidism) corresponding to the first test date].

For example, assuming that a total of three data sets were obtained from a first hypothyroidism patient, that a diagnosis result for the first test was 'hypothyroidism' with a hormone concentration of a first concentration, that a diagnosis result for the second test was 'normal' with a hormone concentration of a second concentration, and that a diagnosis result for the third test was 'normal' with a hormone concentration of a third concentration, training data sets that may be obtained from the clinical data of the first hypothyroidism patient may be generated as follows.
[(a period average heart rate corresponding to a first test date - a period average heart rate corresponding to a second test date), a second concentration, hypothyroidism],
[(the period average heart rate corresponding to the first test date - a period average heart rate corresponding to a third test date), a third concentration, hypothyroidism],
[(the period average heart rate corresponding to the second test date - the period average heart rate corresponding to the first test date), a first concentration, non-hypothyroidism],
[(the period average heart rate corresponding to the second test date - the period average heart rate corresponding to the third test date), the third concentration, non-hypothyroidism],
[(the period average heart rate corresponding to the third test date - the period average heart rate corresponding to the first test date), the first concentration, non-hypothyroidism], and
[(the period average heart rate corresponding to the third test date - the period average heart rate corresponding to the second test date), the second concentration, non-hypothyroidism],

As another example, assuming that a total of four data sets were obtained from a second hypothyroidism patient, that a diagnosis result for the first test was 'hypothyroidism' with a hormone concentration of a first concentration, that a diagnosis result for the second test was `normal' with a hormone concentration of a second concentration, that a diagnosis result for the third test was 'hypothyroidism' with a hormone concentration of a third concentration, and that a diagnosis result for the fourth test was 'normal' with a hormone concentration of a fourth concentration, training data sets that may be obtained from the clinical data of the second hypothyroidism patient may be generated as follows.
[(a period average heart rate corresponding to a first test date - a period average heart rate corresponding to a second test date), a second concentration, hypothyroidism],
[(the period average heart rate corresponding to the first test date - a period average heart rate corresponding to a third test date), a third concentration, hypothyroidism],
[(the period average heart rate corresponding to the first test date - a period average heart rate corresponding to a fourth test date), a fourth concentration, hypothyroidism],
[(the period average heart rate corresponding to the second test date - the period average heart rate corresponding to the first test date), a first concentration, non-hypothyroidism],
[(the period average heart rate corresponding to the second test date - the period average heart rate corresponding to the third test date), the third concentration, non-hypothyroidism],
[(the period average heart rate corresponding to the second test date - the period average heart rate corresponding to the fourth test date), the fourth concentration, non-hypothyroidism],
[(the period average heart rate corresponding to the third test date - the period average heart rate corresponding to the first test date), the first concentration, hypothyroidism],
[(the period average heart rate corresponding to the third test date - the period average heart rate corresponding to the second test date), the second concentration, hypothyroidism],
[(the period average heart rate corresponding to the third test date - the period average heart rate corresponding to the fourth test date), the fourth concentration, hypothyroidism],
[(the period average heart rate corresponding to the fourth test date - the period average heart rate corresponding to the first test date), the first concentration, non-hypothyroidism],
[(the period average heart rate corresponding to the fourth test date - the period average heart rate corresponding to the second test date), the second concentration, non-hypothyroidism], and
[(the period average heart rate corresponding to the fourth test date - the period average heart rate corresponding to the third test date), the third concentration, non-hypothyroidism],

In the first exemplary embodiment, a hormone concentration may be one selected from a group consisting of a concentration of T4, a concentration of free T4, a concentration of T3, a concentration of free T3, a concentration of TSH, and a concentration of thyrotropin-releasing hormone (TRH), or a combination thereof.

### Training Method Embodiment #2. Difference Value from Heart Rates on Reference Date, Period Average Heart Rate on Target Date, and Hormone Value on Reference Date

According to a second exemplary embodiment described in the present application, in order to train a learning model, a value obtained by subtracting, from a period average heart rate corresponding to a first test date, a period average heart rate corresponding to a second test date, a hormone concentration on the first test date, and the period average heart rate corresponding to the second test date may be used as input values, and a diagnosis result corresponding to the first test date may be used as a labeling value.

Herein, as the learning model, classification models including light gradient boosting machine, support vector machine, random forest, extra trees, ada boost, extreme gradient boosting, CatBoost, etc. may be used.

The learning model includes a hyperthyroidism prediction model for predicting hyperthyroidism and a hypothyroidism prediction model for predicting hypothyroidism.

According to the second exemplary embodiment, when the hyperthyroidism prediction model is trained, all the obtained clinical data cannot be used, and only clinical data of patients diagnosed with hyperthyroidism at least once as a diagnosis result may be used as training data.

Hereinafter, for convenience of description, a patient who has been diagnosed with hyperthyroidism at least once as a diagnosis result may be referred to as a 'hyperthyroidism patient'.

In the meantime, in calculating a period average heart rate, resting heart rates for a predetermined period predetermined based on a particular date may be used, wherein the predetermined period may be one of the following: 1, 2, 3, 4, 5, 6, 7, 8, 9, 10, 11, 12, 13, 14, 15, 16, 17, 18, 19, 20, 21, 22, 23, 24, 25, 26, 27, 28, 29, and 30 days.

According to the second exemplary embodiment, in order to train the hyperthyroidism prediction model, clinical data of hyperthyroidism patients may be used to generate training data sets in the format such as [(a period average heart rate corresponding to a first test date - a period average heart rate corresponding to a second test date), the period average heart rate corresponding to the first test date, a thyroid hormone concentration corresponding to the second test date, a diagnosis result (hyperthyroidism or non-hyperthyroidism) corresponding to the first test date].

For example, assuming that a total of three data sets were obtained from a first hyperthyroidism patient, that a diagnosis result for the first test was 'hyperthyroidism' with a hormone concentration of a first concentration, that a diagnosis result for the second test was 'normal' with a hormone concentration of a second concentration, and that a diagnosis result for the third test was 'normal' with a hormone concentration of a third concentration, training data sets that may be obtained from the clinical data of the first hyperthyroidism patient may be generated as follows.
[(a period average heart rate corresponding to a first test date - a period average heart rate corresponding to a second test date), the period average heart rate corresponding to the first test date, a second concentration, hyperthyroidism],
[(the period average heart rate corresponding to the first test date - a period average heart rate corresponding to a third test date), the period average heart rate corresponding to the first test date, a third concentration, hyperthyroidism],
[(the period average heart rate corresponding to the second test date - the period average heart rate corresponding to the first test date), the period average heart rate corresponding to the second test date, a first concentration, non-hyperthyroidism],
[(the period average heart rate corresponding to the second test date - the period average heart rate corresponding to the third test date), the period average heart rate corresponding to the second test date, the third concentration, non-hyperthyroidism],
[(the period average heart rate corresponding to the third test date - the period average heart rate corresponding to the first test date), the period average heart rate corresponding to the third test date, the first concentration, non-hyperthyroidism], and
[(the period average heart rate corresponding to the third test date - the period average heart rate corresponding to the second test date), the period average heart rate corresponding to the third test date, the second concentration, non-hyperthyroidism],

As another example, assuming that a total to four data sets were obtained from a second hyperthyroidism patient, that a diagnosis result for the first test was 'hyperthyroidism' with a hormone concentration of a first concentration, that a diagnosis result for the second test was 'normal' with a hormone concentration of a second concentration, that a diagnosis result for the third test was 'hyperthyroidism' with a hormone concentration of a third concentration, and that a diagnosis result for the fourth test was 'normal' with a hormone concentration of a fourth concentration, training data sets that may be obtained from the clinical data of the second hyperthyroidism patient may be generated as follows.
[(a period average heart rate corresponding to a first test date - a period average heart rate corresponding to a second test date), the period average heart rate corresponding to the first test date, a second concentration, hyperthyroidism],
[(the period average heart rate corresponding to the first test date - a period average heart rate corresponding to a third test date), the period average heart rate corresponding to the first test date, a third concentration, hyperthyroidism],
[(the period average heart rate corresponding to the first test date - a period average heart rate corresponding to a fourth test date), the period average heart rate corresponding to the first test date, a fourth concentration, hyperthyroidism],
[(the period average heart rate corresponding to the second test date - the period average heart rate corresponding to the first test date), the period average heart rate corresponding to the second test date, a first concentration, non-hyperthyroidism],
[(the period average heart rate corresponding to the second test date - the period average heart rate corresponding to the third test date), the period average heart rate corresponding to the second test date, the third concentration, non-hyperthyroidism],
[(the period average heart rate corresponding to the second test date - the period average heart rate corresponding to the fourth test date), the period average heart rate corresponding to the second test date, the fourth concentration, non-hyperthyroidism],
[(the period average heart rate corresponding to the third test date - the period average heart rate corresponding to the first test date), the period average heart rate corresponding to the third test date, the first concentration, hyperthyroidism],
[(the period average heart rate corresponding to the third test date - the period average heart rate corresponding to the second test date), the period average heart rate corresponding to the third test date, the second concentration, hyperthyroidism],
[(the period average heart rate corresponding to the third test date - the period average heart rate corresponding to the fourth test date), the period average heart rate corresponding to the third test date, the fourth concentration, hyperthyroidism],
[(the period average heart rate corresponding to the fourth test date - the period average heart rate corresponding to the first test date), the period average heart rate corresponding to the fourth test date, the first concentration, non-hyperthyroidism],
[(the period average heart rate corresponding to the fourth test date - the period average heart rate corresponding to the second test date), the period average heart rate corresponding to the fourth test date, the second concentration, non-hyperthyroidism], and
[(the period average heart rate corresponding to the fourth test date - the period average heart rate corresponding to the third test date), the period average heart rate corresponding to the fourth test date, the third concentration, non-hyperthyroidism],

According to the second exemplary embodiment, when the hypothyroidism prediction model is trained, all the obtained clinical data cannot be used, and only clinical data of patients diagnosed with hypothyroidism at least once as a diagnosis result may be used as training data.

Hereinafter, for convenience of description, a patient who has been diagnosed with hypothyroidism at least once as a diagnosis result may be referred to as a 'hypothyroidism patient'.

According to the second exemplary embodiment, in order to train the hypothyroidism prediction model, clinical data of hypothyroidism patients may be used to generate training data sets in the format such as [(a period average heart rate corresponding to a first test date - a period average heart rate corresponding to a second test date), the period average heart rate corresponding to the first test date, a thyroid hormone concentration corresponding to the second test date, a diagnosis result (hypothyroidism or non-hypothyroidism) corresponding to the first test date].

For example, assuming that a total of three data sets were obtained from a first hypothyroidism patient, that a diagnosis result for the first test was 'hypothyroidism' with a hormone concentration of a first concentration, that a diagnosis result for the second test was 'normal' with a hormone concentration of a second concentration, and that a diagnosis result for the third test was 'normal' with a hormone concentration of a third concentration, training data sets that may be secured from the clinical data of the first hypothyroidism patient may be generated as follows.
[(a period average heart rate corresponding to the first test date - a period average heart rate corresponding to the second test date), the period average heart rate corresponding to the first test date, a second concentration, hypothyroidism],
[(the period average heart rate corresponding to the first test date - a period average heart rate corresponding to the third test date), the period average heart rate corresponding to the first test date, a third concentration, hypothyroidism],
[(the period average heart rate corresponding to the second test date - the period average heart rate corresponding to the first test date), the period average heart rate corresponding to the second test date, a first concentration, non-hypothyroidism],
[(the period average heart rate corresponding to the second test date - the period average heart rate corresponding to the third test date), the period average heart rate corresponding to the second test date, the third concentration, non-hypothyroidism],
[(the period average heart rate corresponding to the third test date - the period average heart rate corresponding to the first test date), the period average heart rate corresponding to the third test date, the first concentration, non-hypothyroidism], and
[(the period average heart rate corresponding to the third test date - the period average heart rate corresponding to the second test date), the period average heart rate corresponding to the third test date, the second concentration, non-hypothyroidism],

As another example, assuming that a total of four data sets were obtained from a second hypothyroidism patient, that a diagnosis result for the first test was 'hypothyroidism' with a hormone concentration of a first concentration, that a diagnosis result for the second test was 'normal' with a hormone concentration of a second concentration, that a diagnosis result for the third test was 'hypothyroidism' with a hormone concentration of a third concentration, and that a diagnosis result for the fourth test was 'normal' with a hormone concentration of a fourth concentration, training data sets that may be secured from the clinical data of the second hypothyroidism patient may be generated as follows.
[(a period average heart rate corresponding to the first test date - a period average heart rate corresponding to the second test date), the period average heart rate corresponding to the first test date, a second concentration, hypothyroidism],
[(the period average heart rate corresponding to the first test date - a period average heart rate corresponding to the third test date), the period average heart rate corresponding to the first test date, a third concentration, hypothyroidism],
[(the period average heart rate corresponding to the first test date - a period average heart rate corresponding to the fourth test date), the period average heart rate corresponding to the first test date, a fourth concentration, hypothyroidism],
[(the period average heart rate corresponding to the second test date - the period average heart rate corresponding to the first test date), the period average heart rate corresponding to the second test date, a first concentration, non-hypothyroidism],
[(the period average heart rate corresponding to the second test date - the period average heart rate corresponding to the third test date), the period average heart rate corresponding to the second test date, the third concentration, non-hypothyroidism],
[(the period average heart rate corresponding to the second test date - the period average heart rate corresponding to the fourth test date), the period average heart rate corresponding to the second test date, the fourth concentration, non-hypothyroidism],
[(the period average heart rate corresponding to the third test date - the period average heart rate corresponding to the first test date), the period average heart rate corresponding to the third test date, the first concentration, hypothyroidism],
[(the period average heart rate corresponding to the third test date - the period average heart rate corresponding to the second test date), the period average heart rate corresponding to the third test date, the second concentration, hypothyroidism],
[(the period average heart rate corresponding to the third test date - the period average heart rate corresponding to the fourth test date), the period average heart rate corresponding to the third test date, the fourth concentration, hypothyroidism],
[(the period average heart rate corresponding to the fourth test date - the period average heart rate corresponding to the first test date), the period average heart rate corresponding to the fourth test date, the first concentration, non-hypothyroidism],
[(the period average heart rate corresponding to the fourth test date - the period average heart rate corresponding to the second test date), the period average heart rate corresponding to the fourth test date, the second concentration, non-hypothyroidism], and
[(the period average heart rate corresponding to the fourth test date - the period average heart rate corresponding to the third test date), the period average heart rate corresponding to the fourth test date, the third concentration, non-hypothyroidism],

In the second exemplary embodiment, a hormone concentration may be one selected from a group consisting of a concentration of T4, a concentration of free T4, a concentration of T3, a concentration of free T3, a concentration of TSH, and a concentration of thyrotropin-releasing hormone (TRH), or a combination thereof.

### Training Method Embodiment #3. Difference Value from Heart Rates on Reference Date, Hormone Value on Reference Date, and Change in Distribution

According to a third exemplary embodiment described in the present application, in order to train a learning model, a value obtained by subtracting, from a period average heart rate corresponding to a first test date, a period average heart rate corresponding to a second test date, a value obtained by subtracting a standard deviation of a period heart rates corresponding to the second test date from a standard deviation of a period heart rates corresponding to the first test date, and a hormone concentration of the second test date may be used as input values, and a diagnosis result corresponding to the first test date may be used as a labeling value.

Herein, as the learning model, classification models including light gradient boosting machine, support vector machine, random forest, extra trees, ada boost, extreme gradient boosting, CatBoost, etc. may be used.

The learning model includes a hyperthyroidism prediction model for predicting hyperthyroidism and a hypothyroidism prediction model for predicting hypothyroidism.

According to the third exemplary embodiment, when the hyperthyroidism prediction model is trained, all the obtained clinical data cannot be used, and only clinical data of patients diagnosed with hyperthyroidism at least once as a diagnosis result may be used as training data.

Hereinafter, for convenience of description, a patient who has been diagnosed with hyperthyroidism at least once as a diagnosis result may be referred to as a 'hyperthyroidism patient'.

In the meantime, in calculating a period average heart rate, resting heart rates for a predetermined period predetermined based on a particular date may be used, wherein the predetermined period may be one of the following: 1, 2, 3, 4, 5, 6, 7, 8, 9, 10, 11, 12, 13, 14, 15, 16, 17, 18, 19, 20, 21, 22, 23, 24, 25, 26, 27, 28, 29, and 30 days.

According to the third exemplary embodiment, in order to train the hyperthyroidism prediction model, clinical data of hyperthyroidism patients may be used to generate training data sets in the format such as [(a period average heart rate corresponding to a first test date - a period average heart rate corresponding to a second test date), (a standard deviation of a period heart rates corresponding to the first test date - a standard deviation of a period heart rates corresponding to the second test date), a thyroid hormone concentration corresponding to the second test date, a diagnosis result (hyperthyroidism or non-hyperthyroidism) corresponding to the first test date].

For example, assuming that a total of three data sets were obtained from a first hyperthyroidism patient, that a diagnosis result for the first test was 'hyperthyroidism' with a hormone concentration of a first concentration, that a diagnosis result for the second test was 'normal' with a hormone concentration of a second concentration, and that a diagnosis result for the third test was 'normal' with a hormone concentration of a third concentration, training data sets that may be obtained from the clinical data of the first hyperthyroidism patient may be generated as follows.
[(a period average heart rate corresponding to a first test date - a period average heart rate corresponding to a second test date), (a standard deviation of a period heart rates corresponding to the first test date - a standard deviation of a period heart rates corresponding to the second test date), a second concentration, hyperthyroidism],
[(the period average heart rate corresponding to the first test date - a period average heart rate corresponding to a third test date), (the standard deviation of the interval heart rates corresponding to the first test date - a standard deviation of a period heart rates corresponding to the third test date), a third concentration, hyperthyroidism],
[(the period average heart rate corresponding to the second test date - the period average heart rate corresponding to the first test date), (the standard deviation of the interval heart rates corresponding to the second test date - the standard deviation of the interval heart rates corresponding to the first test date), a first concentration, non-hyperthyroidism],
[(the period average heart rate corresponding to the second test date - the period average heart rate corresponding to the third test date), (the standard deviation of the interval heart rates corresponding to the second test date - the standard deviation of the interval heart rates corresponding to the third test date), the third concentration, non-hyperthyroidism],
[(the period average heart rate corresponding to the third test date - the period average heart rate corresponding to the first test date), (the standard deviation of the interval heart rates corresponding to the third test date - the standard deviation of the interval heart rates corresponding to the first test date), the first concentration, non-hyperthyroidism], and
[(the period average heart rate corresponding to the third test date - the period average heart rate corresponding to the second test date), (the standard deviation of the interval heart rates corresponding to the third test date - the standard deviation of the interval heart rates corresponding to the second test date), the second concentration, non-hyperthyroidism],

As another example, assuming that a total to four data sets were obtained from a second hyperthyroidism patient, that a diagnosis result for the first test was 'hyperthyroidism' with a hormone concentration of a first concentration, that a diagnosis result for the second test was 'normal' with a hormone concentration of a second concentration, that a diagnosis result for the third test was 'hyperthyroidism' with a hormone concentration of a third concentration, and that a diagnosis result for the fourth test was 'normal' with a hormone concentration of a fourth concentration, training data sets that may be obtained from the clinical data of the second hyperthyroidism patient may be generated as follows.
[(a period average heart rate corresponding to a first test date - a period average heart rate corresponding to a second test date), (a standard deviation of a period heart rates corresponding to the first test date - a standard deviation of a period heart rates corresponding to the second test date), a second concentration, hyperthyroidism],
[(the period average heart rate corresponding to the first test date - a period average heart rate corresponding to a third test date), (the standard deviation of the interval heart rates corresponding to the first test date - a standard deviation of a period heart rates corresponding to the third test date), a third concentration, hyperthyroidism],
[(the period average heart rate corresponding to the first test date - a period average heart rate corresponding to a fourth test date), (the standard deviation of the interval heart rates corresponding to the first test date - a standard deviation of a period heart rates corresponding to the fourth test date), a fourth concentration, hyperthyroidism],
[(the period average heart rate corresponding to the second test date - the period average heart rate corresponding to the first test date), (the standard deviation of the interval heart rates corresponding to the second test date - the standard deviation of the interval heart rates corresponding to the first test date), a first concentration, non-hyperthyroidism],
[(the period average heart rate corresponding to the second test date - the period average heart rate corresponding to the third test date), (the standard deviation of the interval heart rates corresponding to the second test date - the standard deviation of the interval heart rates corresponding to the third test date), the third concentration, non-hyperthyroidism],
[(the period average heart rate corresponding to the second test date - the period average heart rate corresponding to the fourth test date), (the standard deviation of the interval heart rates corresponding to the second test date - the standard deviation of the interval heart rates corresponding to the fourth test date), the fourth concentration, non-hyperthyroidism],
[(the period average heart rate corresponding to the third test date - the period average heart rate corresponding to the first test date), (the standard deviation of the interval heart rates corresponding to the third test date - the standard deviation of the interval heart rates corresponding to the first test date), the first concentration, hyperthyroidism],
[(the period average heart rate corresponding to the third test date - the period average heart rate corresponding to the second test date), (the standard deviation of the interval heart rates corresponding to the third test date - the standard deviation of the interval heart rates corresponding to the second test date), the second concentration, hyperthyroidism],
[(the period average heart rate corresponding to the third test date - the period average heart rate corresponding to the fourth test date), (the standard deviation of the interval heart rates corresponding to the first test date - the standard deviation of the interval heart rates corresponding to the fourth test date), the fourth concentration, hyperthyroidism],
[(the period average heart rate corresponding to the fourth test date - the period average heart rate corresponding to the first test date), (the standard deviation of the interval heart rates corresponding to the fourth test date - the standard deviation of the interval heart rates corresponding to the first test date), the first concentration, non-hyperthyroidism],
[(the period average heart rate corresponding to the fourth test date - the period average heart rate corresponding to the second test date), (the standard deviation of the interval heart rates corresponding to the fourth test date - the standard deviation of the interval heart rates corresponding to the second test date), the second concentration, non-hyperthyroidism], and
[(the period average heart rate corresponding to the fourth test date - the period average heart rate corresponding to the third test date), (the standard deviation of the interval heart rates corresponding to the fourth test date - the standard deviation of the interval heart rates corresponding to the third test date), the third concentration, non-hyperthyroidism]

According to the third exemplary embodiment, when the hypothyroidism prediction model is trained, all the obtained clinical data cannot be used, and only clinical data of patients diagnosed with hypothyroidism at least once as a diagnosis result may be used as training data.

Hereinafter, for convenience of description, a patient who has been diagnosed with hypothyroidism at least once as a diagnosis result may be referred to as a `hypothyroidism patient'.

According to the third exemplary embodiment, in order to train the hypothyroidism prediction model, clinical data of hypothyroidism patients may be used to generate training data sets in the format such as [(a period average heart rate corresponding to a first test date - a period average heart rate corresponding to a second test date), (a standard deviation of a period heart rates corresponding to the first test date - a standard deviation of a period heart rates corresponding to the second test date), a thyroid hormone concentration corresponding to the second test date, a diagnosis result (hypothyroidism or non-hypothyroidism) corresponding to the first test date].

For example, assuming that a total of three data sets were obtained from a first hypothyroidism patient, that a diagnosis result for the first test was 'hypothyroidism' with a hormone concentration of a first concentration, that a diagnosis result for the second test was 'normal' with a hormone concentration of a second concentration, and that a diagnosis result for the third test was 'normal' with a hormone concentration of a third concentration, training data sets that may be obtained from the clinical data of the first hypothyroidism patient may be generated as follows.
[(a period average heart rate corresponding to a first test date - a period average heart rate corresponding to a second test date), (a standard deviation of a period heart rates corresponding to the first test date - a standard deviation of a period heart rates corresponding to the second test date), a second concentration, hypothyroidism],
[(the period average heart rate corresponding to the first test date - a period average heart rate corresponding to a third test date), (the standard deviation of the interval heart rates corresponding to the first test date - a standard deviation of a period heart rates corresponding to the third test date), a third concentration, hypothyroidism],
[(the period average heart rate corresponding to the second test date - the period average heart rate corresponding to the first test date), (the standard deviation of the interval heart rates corresponding to the second test date - the standard deviation of the interval heart rates corresponding to the first test date), a first concentration, non-hypothyroidism],
[(the period average heart rate corresponding to the second test date - the period average heart rate corresponding to the third test date), (the standard deviation of the interval heart rates corresponding to the second test date - the standard deviation of the interval heart rates corresponding to the third test date), the third concentration, non-hypothyroidism],
[(the period average heart rate corresponding to the third test date - the period average heart rate corresponding to the first test date), (the standard deviation of the interval heart rates corresponding to the third test date - the standard deviation of the interval heart rates corresponding to the first test date), the first concentration, non-hypothyroidism], and
[(the period average heart rate corresponding to the third test date - the period average heart rate corresponding to the second test date), (the standard deviation of the interval heart rates corresponding to the third test date - the standard deviation of the interval heart rates corresponding to the second test date), the second concentration, non-hypothyroidism],

As another example, assuming that a total of four data sets were obtained from a second hypothyroidism patient, that a diagnosis result for the first test was 'hypothyroidism' with a hormone concentration of a first concentration, that a diagnosis result for the second test was 'normal' with a hormone concentration of a second concentration, that a diagnosis result for the third test was 'hypothyroidism' with a hormone concentration of a third concentration, and that a diagnosis result for the fourth test was 'normal' with a hormone concentration of a fourth concentration, training data sets that may be obtained from the clinical data of the second hypothyroidism patient may be generated as follows.
[(a period average heart rate corresponding to a first test date - a period average heart rate corresponding to a second test date), (a standard deviation of a period heart rates corresponding to the first test date - a standard deviation of a period heart rates corresponding to the second test date), a second concentration, hypothyroidism],
[(the period average heart rate corresponding to the first test date - a period average heart rate corresponding to a third test date), (the standard deviation of the interval heart rates corresponding to the first test date - a standard deviation of a period heart rates corresponding to the third test date), a third concentration, hypothyroidism],
[(the period average heart rate corresponding to the first test date - a period average heart rate corresponding to a fourth test date), (the standard deviation of the interval heart rates corresponding to the first test date - a standard deviation of a period heart rates corresponding to the fourth test date), a fourth concentration, hypothyroidism],
[(the period average heart rate corresponding to the second test date - the period average heart rate corresponding to the first test date), (the standard deviation of the interval heart rates corresponding to the second test date - the standard deviation of the interval heart rates corresponding to the first test date), a first concentration, non-hypothyroidism],
[(the period average heart rate corresponding to the second test date - the period average heart rate corresponding to the third test date), (the standard deviation of the interval heart rates corresponding to the second test date - the standard deviation of the interval heart rates corresponding to the third test date), the third concentration, non-hypothyroidism],
[(the period average heart rate corresponding to the second test date - the period average heart rate corresponding to the fourth test date), (the standard deviation of the interval heart rates corresponding to the second test date - the standard deviation of the interval heart rates corresponding to the fourth test date), the fourth concentration, non-hypothyroidism],
[(the period average heart rate corresponding to the third test date - the period average heart rate corresponding to the first test date), (the standard deviation of the interval heart rates corresponding to the third test date - the standard deviation of the interval heart rates corresponding to the first test date), the first concentration, hypothyroidism],
[(the period average heart rate corresponding to the third test date - the period average heart rate corresponding to the second test date), (the standard deviation of the interval heart rates corresponding to the third test date - the standard deviation of the interval heart rates corresponding to the second test date), the second concentration, hypothyroidism],
[(the period average heart rate corresponding to the third test date - the period average heart rate corresponding to the fourth test date), (the standard deviation of the interval heart rates corresponding to the third test date - the standard deviation of the interval heart rates corresponding to the fourth test date), the fourth concentration, hypothyroidism],
[(the period average heart rate corresponding to the fourth test date - the period average heart rate corresponding to the first test date), (the standard deviation of the interval heart rates corresponding to the fourth test date - the standard deviation of the interval heart rates corresponding to the first test date), the first concentration, non-hypothyroidism],
[(the period average heart rate corresponding to the fourth test date - the period average heart rate corresponding to the second test date), (the standard deviation of the interval heart rates corresponding to the fourth test date - the standard deviation of the interval heart rates corresponding to the second test date), the second concentration, non-hypothyroidism], and
[(the period average heart rate corresponding to the fourth test date - the period average heart rate corresponding to the third test date), (the standard deviation of the interval heart rates corresponding to the fourth test date - the standard deviation of the interval heart rates corresponding to the third test date), the third concentration, non-hypothyroidism]

In the above description of the third exemplary embodiment, a difference value of standard deviation is used as an input value. However, any one value selected from a group consisting of the following or a combination thereof may be used as an input value: a difference value in standard deviation of period heart rates; a difference value in relative standard deviation of period heart rates; a difference value in skewness of period heart rates; a difference value in kurtosis of period heart rates; and a JS divergence indicating a difference between a distribution of period heart rates corresponding to an X-th test date and a distribution of period heart rates corresponding to an Y-th test date. These in the group are calculated with respect to a relation between the period heart rates corresponding to the X-th test date and the period heart rates corresponding to the Y-th test date.

### Training Method Embodiment #4. Difference Value from Heart Rates on Reference Date, Period Average Heart Rate on Target Date, Hormone Value on Reference Date, and Change in Distribution

According to a fourth exemplary embodiment described in the present application, in order to train a learning model, a value obtained by subtracting, from a period average heart rate corresponding to a first test date, a period average heart rate corresponding to a second test date, a value obtained by subtracting a standard deviation of period heart rates corresponding to the second test date from a standard deviation of period heart rates corresponding to the first test date, the period average heart rate corresponding to the first test date, and a hormone concentration of the second test date may be used as input values, and a diagnosis result corresponding to the first test date may be used as a labeling value.

Compared to the third exemplary embodiment, except that "a period average heart rate on a first test date" is further used as an input value, the fourth exemplary embodiment is almost the same as the third exemplary embodiment in other respects, so a detailed description of the fourth exemplary embodiment will be omitted.

In the above description of the fourth exemplary embodiment, a difference value of standard deviation is used as an input value. However, any one value selected from a group consisting of the following or a combination thereof may be used as an input value: a difference value in standard deviation of period heart rates; a difference value in relative standard deviation of period heart rates; a difference value in skewness of period heart rates; a difference value in kurtosis of period heart rates; and a JS divergence indicating a difference between a distribution of period heart rates corresponding to an X-th test date and a distribution of period heart rates corresponding to an Y-th test date. These in the group are calculated with respect to a relation between the period heart rates corresponding to the X-th test date and the period heart rates corresponding to the Y-th test date.

### Training Method Embodiment #5. Rate of Change in Heart Rates on Reference Date, and Hormone Value on Reference Date

According to a fifth exemplary embodiment described in the present application, in order to train a learning model, a rate of change in a period average heart rate on a first test date with respect to a second test date (that is, (the period average heart rate corresponding to the first test date - the period average heart rate corresponding to the second test date) / (the period average heart rate corresponding to the second test date)), and a hormone concentration of the second test date may be used as input values, and a diagnosis result corresponding to the first test date may be used as a labeling value.

Compared to the first exemplary embodiment, except that "a rate of change in a period average heart rate" is used instead of "an amount of change in a period average heart rate", the fifth exemplary embodiment is almost the same as the first exemplary embodiment in other respects, so a detailed description of the fifth exemplary embodiment will be omitted.

### Training Method Embodiment #6. Rate of Change in Heart Rates on Reference Date, Period Average Heart Rate on Target Date, and Hormone Value on Reference Date

According to a sixth exemplary embodiment described in the present application, in order to train a learning model, a rate of change in a period average heart rate on a first test date with respect to a second test date (that is, (the period average heart rate corresponding to the first test date - the period average heart rate corresponding to the second test date) / (the period average heart rate corresponding to the second test date)), the period average heart rate corresponding to the first test date, and a hormone concentration on the second test date may be used as input values, and a diagnosis result corresponding to the first test date may be used as a labeling value.

Compared to the fifth exemplary embodiment, except that "a period average heart rate on a first test date" is further used as an input value, the sixth exemplary embodiment is almost the same as the fifth exemplary embodiment in other respects, so a detailed description of the sixth exemplary embodiment will be omitted.

### Training Method Embodiment #7. Rate of Change in Heart Rates on Reference Date, Hormone Value on Reference Date, and Change in Distribution

According to a seventh exemplary embodiment described in the present application, in order to train a learning model, a rate of change in a period average heart rate on a first test date with respect to a second test date (that is, (the period average heart rate corresponding to the first test date - the period average heart rate corresponding to the second test date) / (the period average heart rate corresponding to the second test date)), an amount of change in a relative standard deviation of period heart rates on the first test date with respect to the second test date (that is, (a standard deviation of period heart rates corresponding to the first test date / the period average heart rate corresponding to the first test date) - (a standard deviation of period heart rates corresponding to the second test date / the period average heart rate corresponding to the second test date)), and a hormone concentration on the second test date may be used as input values, and a diagnosis result corresponding to the first test date may be used as a labeling value.

Compared to the fifth exemplary embodiment, except that "an amount of change in a relative standard deviation of interval heart rates" is further used as an input value, the seventh exemplary embodiment is almost the same as the fifth exemplary embodiment in other respects, so a detailed description of the seventh exemplary embodiment will be omitted.

In the above description of the seventh exemplary embodiment, a difference value in relative standard deviation is used as an input value. However, any one value selected from a group consisting of the following or a combination thereof may be used as an input value: a difference value in standard deviation of period heart rates; a difference value in relative standard deviation of period heart rates; a difference value in skewness of period heart rates; a difference value in kurtosis of period heart rates; and a JS divergence indicating a difference between a distribution of period heart rates corresponding to an X-th test date and a distribution of period heart rates corresponding to an Y-th test date. These in the group are calculated with respect to a relation between the period heart rates corresponding to the X-th test date and the period heart rates corresponding to the Y-th test date

### Training Method Embodiment #8. Rate of Change in Heart Rates on Reference Date, Period Average Heart Rate on Target Date, Hormone Value on Reference Date, and Change in Distribution

According to an eighth exemplary embodiment described in the present application, in order to train a learning model, a rate of change in a period average heart rate on a first test date with respect to a second test date (that is, (the period average heart rate corresponding to the first test date - the period average heart rate corresponding to the second test date) / (the period average heart rate corresponding to the second test date)), an amount of change in a relative standard deviation of interval heart rates on the first test date with respect to the second test date (that is, (a standard deviation of period heart rates corresponding to the first test date / the period average heart rate corresponding to the first test date) - (a standard deviation of period heart rates corresponding to the second test date / the period average heart rate corresponding to the second test date)), the period average heart rate corresponding to the first test date, and a hormone concentration on the second test date may be used as input values, and a diagnosis result corresponding to the first test date may be used as a labeling value.

Compared to the seventh exemplary embodiment, except that "a period average heart rate on a first reference date" is further used as an input value, the eighth exemplary embodiment is almost the same as the seventh exemplary embodiment in other respects, so a detailed description of the eighth exemplary embodiment will be omitted.

In the above description of the eighth exemplary embodiment, a difference value in relative standard deviation is used as an input value. However, any one value selected from a group consisting of the following or a combination thereof may be used as an input value: a difference value in standard deviation of period heart rates; a difference value in relative standard deviation of period heart rates; a difference value in skewness of period heart rates; a difference value in kurtosis of period heart rates; and a JS divergence indicating a difference between a distribution of period heart rates corresponding to an X-th test date and a distribution of period heart rates corresponding to an Y-th test date. These in the group are calculated with respect to a relation between the period heart rates corresponding to the X-th test date and the period heart rates corresponding to the Y-th test date.

### Training Method Embodiment #9. Rate of Change (or Amount of Change) in Heart Rates on Reference Date, Hormone Value on Reference Date, Change in Distribution, and Day Gap between Test Dates

According to a ninth exemplary embodiment described in the present application, in order to train a learning model, a rate of change in a period average heart rate on a first test date with respect to a second test date (that is, (the period average heart rate corresponding to the first test date - the period average heart rate corresponding to the second test date) / (the period average heart rate corresponding to the second test date)), an amount of change in a relative standard deviation of period heart rates on the first test date with respect to the second test date (that is, (a standard deviation of period heart rates corresponding to the first test date / the period average heart rate corresponding to the first test date) - (a standard deviation of period heart rates corresponding to the second test date / the period average heart rate corresponding to the second test date)), a hormone concentration on the second test date, and a day gap (days) between the first test date and the second test date may be used as input values, and a diagnosis result corresponding to the first test date may be used as a labeling value.

Compared to the seventh exemplary embodiment, except that a day gap between a first test date and a second test date is further used as an input value, the ninth exemplary embodiment is almost the same as the seventh exemplary embodiment, so a detailed description of the ninth exemplary embodiment will be omitted.

In the above description of the ninth exemplary embodiment, a difference value in relative standard deviation is used as an input value. However, any one value selected from a group consisting of the following or a combination thereof may be used as an input value: a difference value in standard deviation of period heart rates; a difference value in relative standard deviation of period heart rates; a difference value in skewness of period heart rates; a difference value in kurtosis of period heart rates; and a JS divergence indicating a difference between a distribution of period heart rates corresponding to an X-th test date and a distribution of period heart rates corresponding to an Y-th test date. These in the group are calculated with respect to a relation between the period heart rates corresponding to the X-th test date and the period heart rates corresponding to the Y-th test date.

In the meantime, instead of using a rate of change in heart rates on a reference date and an amount of change in a relative standard deviation, an amount of change in heart rates on a reference date and an amount of change in a standard deviation may be used. In this case, in addition to an amount of change in a relative standard deviation, any one value selected from a group consisting of the following or a combination thereof may be used as an input value: a difference value in standard deviation of period heart rates; a difference value in relative standard deviation of period heart rates; a difference value in skewness of period heart rates; a difference value in kurtosis of period heart rates; and a JS divergence indicating a difference between a distribution of period heart rates corresponding to an X-th test date and a distribution of period heart rates corresponding to an Y-th test date. These in the group are calculated with respect to a relation between the period heart rates corresponding to the X-th test date and the period heart rates corresponding to the Y-th test date.

### Training Method Embodiment #10. Combination of Variables

According to a 10th exemplary embodiment described in the present application, in order to train a learning model, a combination value of variables shown in [Table 1] below may be further used as an input value.

**[Table 1]**

| No | Variables |
|---|---|
| 1 | Amount of change in period average heart rate |
| 2 | Rate of change in period average heart rate |
| 3 | Amount of change in standard deviation |
| 4 | Amount of change in relative standard deviation |
| 5 | Amount of change in skewness of period heart rates |
| 6 | Amount of change in kurtosis of period heart rates |
| 7 | JS divergence |
| 8 | TSH concentration on second reference date |
| 9 | Free T4 concentration on second reference date |
| 10 | T4 concentration on second reference date |
| 11 | Free T3 concentration on second reference date |
| 12 | T3 concentration on second reference date |
| 13 | TRH concentration on second reference date |

When a first period average heart rate, a first standard deviation, a first skewness, a first kurtosis, a first TSH hormone concentration, a first free T4 concentration, a first T4 concentration, a first free T3 concentration, a first T3 concentration, a first TRH concentration, and a diagnosis result for thyroid dysfunction with respect to a first reference date correspond to the first reference date, and a second period average heart rate, a second standard deviation, a second skewness, a second kurtosis, a second TSH hormone concentration, a second free T4 concentration, a second T4 concentration, a second free T3 concentration, a second T3 concentration, a second TRH concentration, and a diagnosis result for thyroid dysfunction with respect to a second reference date correspond to the second reference date, the variables are calculated as follows.
(1) An amount of change in a period average heart rate = the period average heart rate on the second reference date - the period average heart rate on the first reference date
(2) A rate of change in a period average heart rate = (the period average heart rate on the second reference date - the period average heart rate on the first reference date) / the period average heart rate on the second reference date
(3) An amount of change in a standard deviation = the standard deviation of period heart rates on the second reference date - the standard deviation of period heart rates on the first reference date
(4) An amount of change in a relative standard deviation = (the standard deviation of period heart rates on the second reference date / the period average heart rate on the second reference date) - (the standard deviation of period heart rates on the first reference date / the period average heart rate on the first reference date)
(5) An amount of change in skewness of period heart rates = the skewness of period heart rates on the second reference date - the skewness of period heart rates on the first reference date
(6) An amount of change in kurtosis of period heart rates = the kurtosis of period heart rates on the second reference date - the skewness of period heart rates on the first reference date
(7) JS divergence : a JS divergence calculated between period heart rates on the second reference date and periodl heart rates on the first reference date

Herein, a combination value of the variables means one of the following: a value obtained by multiplying one value (hereinafter, referred to as a first value) selected among the variables, by another value (hereinafter, referred to as a second value) selected among the variables; a value obtained by dividing the first value by the second value; and a value obtained by dividing the second value by the first value. For example, the combination value may be a value obtained by dividing a free T4 concentration value on the second reference date by a JS divergence.

In theory, the number of combination values may be 12*11 = 132.

In the meantime, two or more combination values of the variables may be used.

When the learning model is trained according to the 10th exemplary embodiment, input values may be as follows.
[an amount of change in a period average heart rate, at least one of the hormone concentrations on a second test date, at least one of the above-described combination values]
[an amount of change in a period average heart rate, at least one of the hormone concentrations on a second test date, an amount of change in a standard deviation of a period average heart rate, at least one of the above-described combination values]
[an amount of change in a period average heart rate, at least one of the hormone concentrations on a second test date, a period average heart rate on a first reference date, at least one of the above-described combination values]
[an amount of change in a period average heart rate, at least one of the hormone concentrations on a second test date, an amount of change in a standard deviation of a period average heart rate, a period average heart rate on a first reference date, at least one of the above-described combination values]
[an amount of change in a period average heart rate, at least one of the hormone concentrations on a second test date, an amount of change in a standard deviation of a period average heart rate, a period average heart rate on a first reference date, a day gap between the first reference date and a second reference date, at least one of the above-described combination values]
[a rate of change in a period average heart rate, at least one of the hormone concentrations on a second test date, at least one of the above-described combination values]
[a rate of change in a period average heart rate, at least one of the hormone concentrations on a second test date, an amount of change in a relative standard deviation of a period average heart rate, at least one of the above-described combination values]
[a rate of change in a period average heart rate, at least one of the hormone concentrations on a second test date, a period average heart rate on a first reference date, at least one of the above-described combination values]
[a rate of change in a period average heart rate, at least one of the hormone concentrations on a second test date, an amount of change in a relative standard deviation of a period average heart rate, a period average heart rate on a first reference date, at least one of the above-described combination values]
[a rate of change in a period average heart rate, at least one of the hormone concentrations on a second test date, an amount of change in a relative standard deviation of a period average heart rate, a period average heart rate on a first reference date, a day gap between the first reference date and a second reference date, at least one of the above-described combination values]
[an amount of change in a period average heart rate, at least one of the hormone concentrations on a second test date, an amount of change in skewness or kurtosis of period average heart rates or a JS divergence, at least one of the above-described combination values]
[an amount of change in a period average heart rate, at least one of the hormone concentrations on a second test date, an amount of change in skewness or kurtosis of period average heart rates or a JS divergence, a period average heart rate on a first reference date, at least one of the above-described combination values]
[an amount of change in a period average heart rate, at least one of the hormone concentrations on a second test date, an amount of change in skewness or kurtosis of period average heart rates or a JS divergence, a period average heart rate on a first reference date, a day gap between the first reference date and a second reference date, at least one of the above-described combination values]
[a rate of change in a period average heart rate, at least one of the hormone concentrations on a second test date, an amount of change in skewness or kurtosis of period average heart rates or a JS divergence, at least one of the above-described combination values]
[a rate of change in a period average heart rate, at least one of the hormone concentrations on a second test date, an amount of change in skewness or kurtosis of period average heart rates or a JS divergence, a period average heart rate on a first reference date, at least one of the above-described combination values]
[a rate of change in a period average heart rate, at least one of the hormone concentrations on a second test date, an amount of change in skewness or kurtosis of period average heart rates or a JS divergence, a period average heart rate on a first reference date, a day gap between the first reference date and a second reference date, at least one of the above-described combination values]
[an amount of change in a period average heart rate, at least one of the hormone concentrations on a second test date, an amount of change in a standard deviation of period average heart rates, an amount of change in skewness or kurtosis of period average heart rates or a JS divergence, at least one of the above-described combination values]
[an amount of change in a period average heart rate, at least one of the hormone concentrations on a second test date, an amount of change in a standard deviation of period average heart rates, an amount of change in skewness or kurtosis of period average heart rates or a JS divergence, a period average heart rate on a first reference date, at least one of the above-described combination values]
[an amount of change in a period average heart rate, at least one of the hormone concentrations on a second test date, an amount of change in a standard deviation of period average heart rates, an amount of change in skewness or kurtosis of period average heart rates or a JS divergence, a period average heart rate on a first reference date, a day gap between the first reference date and a second reference date, at least one of the above-described combination values]
[a rate of change in a period average heart rate, at least one of the hormone concentrations on a second test date, an amount of change in a relative standard deviation of period average heart rates, an amount of change in skewness or kurtosis of period average heart rates or a JS divergence, at least one of the above-described combination values]
[a rate of change in a period average heart rate, at least one of the hormone concentrations on a second test date, an amount of change in a relative standard deviation of period average heart rates, an amount of change in skewness or kurtosis of period average heart rates or a JS divergence, a period average heart rate on a first reference date, at least one of the above-described combination values]
[a rate of change in a period average heart rate, at least one of the hormone concentrations on a second test date, an amount of change in a relative standard deviation of period average heart rates, an amount of change in skewness or kurtosis of period average heart rates or a JS divergence, a period average heart rate on a first reference date, a day gap between the first reference date and a second reference date, at least one of the above-described combination values]

In the meantime, when the learning model is trained according to the 10th exemplary embodiment, a labeling value may be a diagnosis result on a first test date. For example, in the case in which the hyperthyroidism prediction model is trained, when the diagnosis result on the first test date is 'hyperthyroidism', the label value may be labelled as `hyperthyroidism', or when the diagnosis result on the first test date is 'normal', the label value may be labelled as 'non-hyperthyroidism'. In the case in which the hypothyroidism prediction model is trained, when the diagnosis result on the first test date is 'hypothyroidism', the label value may be labelled as 'hypothyroidism', or when the diagnosis result on the first test date is 'normal', the label value may be labelled as 'non-hypothyroidism'.

### Training Method Embodiment #11. Use of Personal Information such as Sex, Age, Etc.

According to an 11th exemplary embodiment described in the present application, in order to train a learning model, in addition to the input values described above in the first exemplary embodiment to the 10th exemplary embodiment, personal information of a subject may be used as an input value. For example, a subject's sex and/or age may be used as an input value.

For example, when a subject's sex and age are used as input values in addition to the input values described in the first exemplary embodiment, a rate of change in a period average heart rate on a first test date with respect to a second test date (that is, (the period average heart rate corresponding to the first test date - the period average heart rate corresponding to the second test date) / (the period average heart rate corresponding to the second test date)), the period average heart rate corresponding to the first test date, a hormone concentration on the second test date, a sex, and an age may be used as input values, and a diagnosis result corresponding to the first test date may be used as a labeling value.

A detailed description of the further use of personal information of a subject in addition to the input values described in the first exemplary embodiment to the 10th exemplary embodiment will be omitted.

In the meantime, although it has been described that both a sex and an age are used as a subject's personal information, an embodiment in which only a sex or an age is used instead of using a sex and an age together may be possible, and other types of personal information other than a sex and an age may be further used.

Hereinafter, described will be a method and a system for predicting a subject's thyroid dysfunction by using a prediction model described in the present application.

### 3. Whole System

### (1) Hardware Construction of System

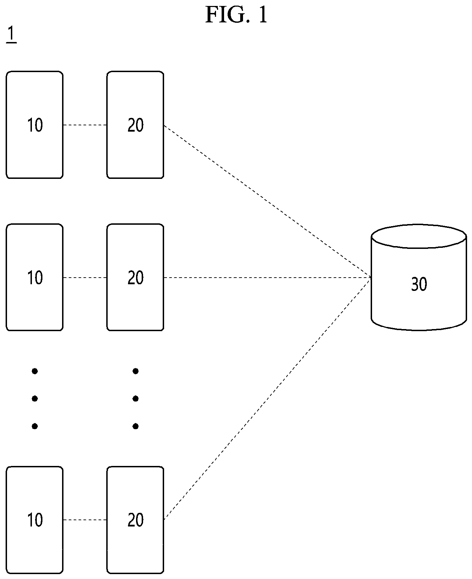

FIG. 1 is a diagram illustrating a system for predicting thyroid dysfunction according to an embodiment described in the present application.

Referring to FIG. 1, the system 1 includes a plurality of heart rate measuring devices 10, a plurality of user terminals 20, and a server 30.

Hereinafter, the plurality of heart rate measuring devices 10, the plurality of user terminals 20, and the server 30 will be described in detail.

### (2) Functions of Heart Rate Measuring Device

The plurality of heart rate measuring devices 10 may measure heart rates of subjects (users). For example, the heart rate measuring devices 10 may measure subjects' heart rates per unit time (beats per minute). That is, the heart rate measuring devices 10 may measure heart rates per minute of subject.

The plurality of heart rate measuring devices 10 may store the measured heart rates of the subjects. Herein, when storing the plurality of heart rates, the heart rate measuring devices 10 match the time points at which the heart rates (heart rates per unit time) are measured thereto, and store the time points together.

The plurality of heart rate measuring devices 10 may transmit the stored heart rates to the plurality of user terminals 20 and/or the server 30.

The plurality of heart rate measuring devices 10 may receive prediction results for thyroid dysfunction from the server 30 or the user terminals 20.

### (3) Type of Heart Rate Measuring Device

The heart rate measuring devices may be wearable watches, wearable bands, or patch-type devices.

### (3) Functions of User Terminal

The plurality of user terminals 20 transmit information to the server 30 over various networks, and receive information from the server 30.

The plurality of user terminals 20 may receive the measured heart rates from the heart rate measuring devices 10, and may store the received heart rates.

The plurality of user terminals 20 may preprocess the stored heart rates. As an example, the user terminals 20 may select heart rates measured during resting periods among all the stored heart rates. As another example, the user terminals 20 may select the above-described period heart rates on the basis of particular dates among all the stored heart rates. As still another example, the user terminals 20 may calculate averages of the period heart rates selected on the basis of the particular dates. As still another example, the user terminals 20 may calculate standard deviations, relative standard deviations, skewness, kurtosis, etc. of the period heart rates selected on the basis of the particular dates.

The plurality of user terminals 20 may obtain test dates for hormone concentrations of the users, and may obtain test results (for example, hormone concentrations and/or doctors' diagnosis results) corresponding to the test dates. The plurality of user terminals 20 may obtain the test dates and the test results corresponding thereto, by the users' input or by receiving the test dates and the test results corresponding thereto from the outside over a network.

The plurality of user terminals 20 may transmit the stored heart rates and/or the preprocessed results to the server 30.

The plurality of user terminals 20 may transmit the test dates and the test results to the server 30.

The plurality of user terminals 20 may receive, from the server 30, prediction results for thyroid dysfunction processed by the server 30.

### (4) Type of User Terminal

The plurality of user terminals 20 may be at least one of a smartphone, a tablet PC, a laptop, and a desktop.

### (5) Functions of Server

The server 30 transmits information to the plurality of user terminals 20 over various networks, and receives information from the plurality of user terminals 20.

The server 30 may receive all or some of the measured heart rates from the plurality of user terminals 20. Herein, the server 30 may preprocess the received heart rates.

Alternatively, the server 30 may receive, from the plurality of user terminals 20, results preprocessed by the user terminals 20.

In addition, the server 30 may acquire, for each user, a test date for a hormone concentration and a test result corresponding thereto. The test dates and the test results may be received from the user terminals 20 or other external devices.

The server 30 may obtain, on the basis of the preprocessed results, prediction results for thyroid dysfunction for subjects.

The server 30 may transmit the prediction results for thyroid dysfunction to the heart rate measuring devices 10 and/or the plurality of user terminals 20.

### (6) Software Construction of System

In order for the system 1 to operate, several software constructions are required.

To perform communication between the user terminals 20 and the server 30, terminal software needs to be installed on the plurality of user terminals 20, and server software needs to be installed on the server 30.

A plurality of prediction models for predicting thyroid dysfunction on the basis of the preprocessed results may be used.

The plurality of prediction models may be run by the software installed on the server 30. Alternatively, the plurality of prediction models may be run by the terminal software installed on the user terminals 20. Alternatively, some of the plurality of learning models may be executed by the user terminals 20, and the others may be executed by the server 30.

### (7) Elements of Heart Rate Measuring Device

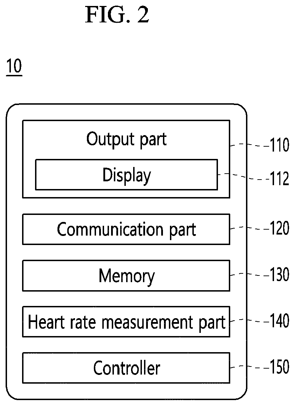

FIG. 2 is a block diagram illustrating a heart rate measuring device described in the present application.

Referring to FIG. 2, a heart rate measuring device 10 described in the present application includes an output part 110, a communication part 120, a memory 130, a heart rate measurement part 140, and a controller 150.

The output part 110 outputs various types of information according to control commands of the controller 150. According to an embodiment, the output part 110 may include a display 112 for outputting information visually to a user. Alternatively, although not shown in the drawings, a speaker for outputting information audibly to a user, and a vibration motor for outputting information tactually to a user may be included.

The communication part 120 may include a wireless communication module and/or a wired communication module. Herein, examples of the wireless communication module may include a Wi-Fi communication module, a cellular communication module, etc.

The memory 130 stores therein executable code readable by the controller 150, processed result values, necessary data, etc. Examples of the memory 130 may include a hard disk drive (HDD), a solid state disk (SSD), a silicon disk drive (SDD), ROM, RAM, etc. The memory 130 may store therein the above-described terminal software, and may store therein executable codes for realizing the above-described various preprocessing algorithms and/or learning models. Furthermore, the memory 130 may store therein a subject's heart rates acquired through the heart rate measurement part 140 and the time points at which the heart rates are measured.

The heart rate measurement part 140 may measure a subject(user)'s heart rate per minute. A method of measuring a subject's heart rate per minute is widely known, so a detailed description thereof will be omitted.

The controller 150 may include at least one processor. Herein, each of the processors may perform a predetermined operation by executing at least one instruction stored in the memory 130. Specifically, the controller 150 may process information according to the terminal software, the preprocessing algorithms, and/or the learning models running on the heart rate measuring device 10. In the meantime, the controller 150 controls the overall operation of the heart rate measuring device 10.

Although not shown in the drawings, the heart rate measuring device 10 may include a user input part. The heart rate measuring device 10 may receive, from a user, various types of information required for the operation of the heart rate measuring device 10 through the user input part.

### (8) Elements of User Terminal

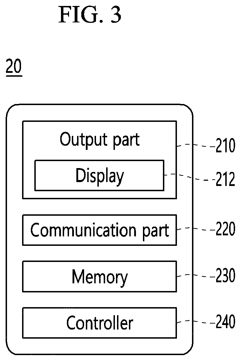

FIG. 3 is a block diagram illustrating a user terminal described in the present application.

Referring to FIG. 3, a user terminal 20 described in the present application includes an output part 210, a communication part 220, a memory 230, and a controller 250.

The output part 210 outputs various types of information according to control commands of the controller 250. According to an embodiment, the output part 210 may include a display 212 for outputting information visually to a user. Alternatively, although not shown in the drawings, a speaker for outputting information audibly to a user, and a vibration motor for outputting information tactually to a user may be included.

The communication part 220 may include a wireless communication module and/or a wired communication module. Herein, examples of the wireless communication module may include a Wi-Fi communication module, a cellular communication module, etc.

The memory 230 stores therein executable code readable by the controller 250, processed result values, necessary data, etc. Examples of the memory 230 may include a hard disk drive (HDD), a solid state disk (SSD), a silicon disk drive (SDD), ROM, RAM, etc. The memory 230 may store therein the above-described terminal software, and may store therein executable codes for realizing the above-described various preprocessing algorithms and/or learning models.

The controller 240 may include at least one processor. Herein, each of the processors may perform a predetermined operation by executing at least one instruction stored in the memory 230. Specifically, the controller 240 may process information according to the terminal software, the preprocessing algorithms, and/or the learning models running on the user terminal 20. In the meantime, the controller 240 controls the overall operation of the user terminal 20.

Although not shown in the drawings, the user terminal 20 may include a user input part. The user terminal 20 may receive, from a user, various types of information required for the operation of the user terminal 20 through the user input part.

### (9) Elements of server

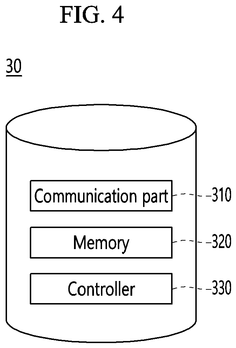

FIG. 4 is a block diagram illustrating a server described in the present application.

Referring to FIG. 4, a server 30 described in the present application includes a communication part 310, a memory 320, and a controller 330.

The communication part 310 may include a wireless communication module and/or a wired communication module. Herein, examples of the wireless communication module may include a Wi-Fi communication module, a cellular communication module, etc.

The memory 320 stores therein executable code readable by the controller 330, processed result values, necessary data, etc. Examples of the memory 320 may include a hard disk drive (HDD), a solid state disk (SSD), a silicon disk drive (SDD), ROM, RAM, etc. The memory 320 may store therein the above-described server software, and may store therein executable codes for realizing the above-described various preprocessing algorithms and/or learning models. Furthermore, the memory 320 may store therein heart rates for each user and/or pre-processing results corresponding thereto from the user terminal 20.

The controller 330 may include at least one processor. Herein, each of the processors may perform a predetermined operation by executing at least one instruction stored in the memory 320. Specifically, the controller 330 may process information according to the server software, the preprocessing algorithms, and/or learning models running on the server 30. In the meantime, the controller 330 controls the overall operation of the server 30.

### 4. Method for Predicting Thyroid Dysfunction

The method for predicting thyroid dysfunction, which is described in the present application, may be performed by the above-described system 1.

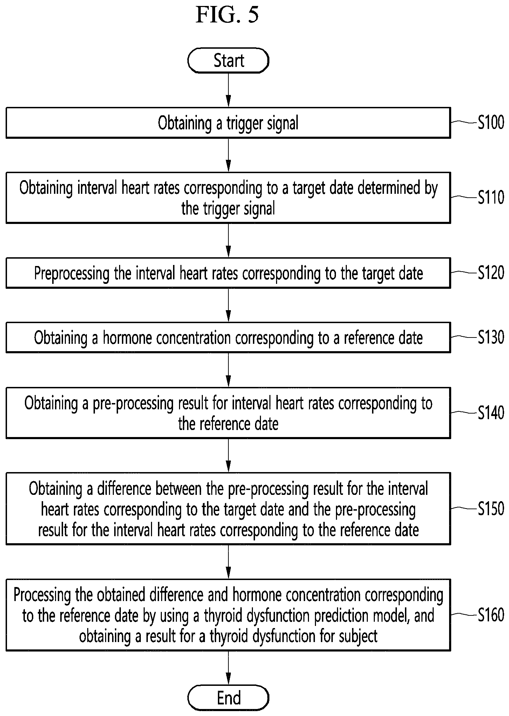

FIG. 5 is a flowchart illustrating a method for predicting thyroid dysfunction described in the present application.

Referring to FIG. 5, the method, which is described in the present application, for predicting thyroid dysfunction for a subject includes: obtaining a trigger signal in step S100; obtaining interval heart rates corresponding to a target date determined by the trigger signal, in step S110; preprocessing the interval heart rates corresponding to the target date in step S120; obtaining a hormone concentration corresponding to a reference date in step S130; obtaining a pre-processing result for interval heart rates corresponding to the reference date in step S140; obtaining a difference between the pre-processing result for the interval heart rates corresponding to the target date and the pre-processing result for the interval heart rates corresponding to the reference date in step S150; and processing the obtained difference and the hormone concentration corresponding to the reference date by using a thyroid dysfunction prediction model, and obtaining a result for a thyroid dysfunction for the subject, in step S160.

### The obtaining of the trigger signal in step S100

The trigger signal may be obtained in step S100.

Herein, the trigger signal refers to a signal for requesting acquisition of a prediction result for thyroid dysfunction for a subject.

The trigger signal may be generated by the user's input. For example, the user may make a request, through the user input part of the user terminal 20, for acquisition of a prediction result for thyroid dysfunction, and in response to the user's request, the user terminal 20 may generate the trigger signal. As another example, the user may make a request, through the user input part of the heart rate measuring device 10 communicating with the user terminal 20, for acquisition of a prediction result for thyroid dysfunction, and in response to the user's request, the heart rate measuring device 10 or the user terminal 20 may generate the trigger signal.

Alternatively, the trigger signal may be automatically generated by the system 1 regardless of a user's request. For example, the software installed on the heart rate measuring device 10, the user terminal 20, or the server 30 may generate the trigger signal according to a predetermined rule. When the trigger signal is generated by the heart rate measuring device 10 or the server 30, the generated trigger signal may be transmitted to the user terminal 20.

### Determining the target date on the basis of the trigger signal in step S110

The target date may be determined by the trigger signal. For example, the date on which the trigger signal is generated may be determined as the target date. As another example, when the trigger signal is generated, a specific day may be designated by the user's input or the system 1. In this case, the designated specific day may be determined as the target date.

### The obtaining of the interval heart rates corresponding to the target date in step S110

The interval heart rates corresponding to the target date may be obtained according to a predetermined period in step S110.

The predetermined period may be one of the following: 1, 2, 3, 4, 5, 6, 7, 8, 9, 10, 11, 12, 13, 14, 15, 16, 17, 18, 19, 20, 21, 22, 23, 24, 25, 26, 27, 28, 29, and 30 days.

When the predetermined period is 10 days and the target date is July 21, the interval heart rates are obtained including resting heart rates corresponding to July 21, resting heart rates corresponding to July 20, resting heart rates corresponding to July 19, resting heart rates corresponding to July 18, resting heart rates corresponding to July 17, resting heart rates corresponding to July 16, resting heart rates corresponding to July 15, resting heart rates corresponding to July 14, resting heart rates corresponding to July 13, and resting heart rates corresponding to July 12.

The interval heart rates are selected among all the heart rates stored corresponding to the dates determined by the determined target date and the predetermined period. On the basis of the measurement time points corresponding to the heart rates, the heart rates for which the measurement time points are within a resting period of the subject (namely, a user) are selected as the resting heart rates.

The resting period may be determined by various methods.

For example, it may be determined the resting period is a period during which the user's movement is equal to or less than a reference value through a motion sensor built in the heart rate measuring device 10 or the user terminal 20.

As another example, it may be determined the resting period is a period during which the user is considered to be resting or sleeping through various sensors built in the heart rate measuring device 10 or the user terminal 20.

As still another example, the resting period may be determined by a sleep period (a sleep start time point and a sleep end time point) that the user inputs in person through the heart rate measuring device 10 or the user terminal 20.

### The preprocessing of the interval heart rates corresponding to the target date in step S120

The interval heart rates corresponding to the target date may be preprocessed in step S120.

An average of the interval heart rates corresponding to the target date may be calculated.

Various parameters related to the **distribution** of the interval heart rates corresponding to the target date may be calculated. As an example, a standard deviation of the interval heart rates corresponding to the target date may be calculated. As another example, the skewness of the interval heart rates corresponding to the target date may be calculated. As still another example, the kurtosis of the interval heart rates corresponding to the target date may be calculated.

Among various pre-processing results of the interval heart rates corresponding to the target date, only the pre-processing results related to the input values used in training the prediction model to be described later may be obtained. For example, when a rate of change in an average value and an amount of change in a relative standard deviation of interval heart rates with respect to a target date and a reference date are used in training the prediction model, only the average and standard deviation of the interval heart rates corresponding to the target date may be obtained from the interval heart rates corresponding to the target date. However, all or some of the above-described pre-processing results may be obtained regardless of the input values used in training the prediction model.

### Determining of the reference date in step S130

The reference date for the subject may be determined in step S130.

The reference date may be selected among one or more test dates on which the subject had a hormone test. That is, the reference date may be selected among past hormone test dates of the subject.

The reference date may be selected among one or more dates stored corresponding to the subject's hormone concentrations. That is, the reference date may be selected among dates for which the subject's past hormone concentrations are stored.

Only one reference date may be selected, but no limitation thereto is imposed. That is, a plurality of the reference dates may be selected.

When only one reference date is selected among a plurality of test dates or a plurality of dates for which hormone concentrations are stored, it may be determined that the reference date is the closest date to the target date.

### Obtaining of the hormone concentration corresponding to the reference date in step S130

The hormone concentration corresponding to the determined reference date may be obtained.

The hormone concentration may be one selected from a group consisting of a T4 concentration, a free T4 concentration, a T3 concentration, a free T3 concentration, a TSH concentration, and a TRH concentration, or may be a combination thereof.

Among the hormone concentrations corresponding to the determined reference date, only the hormone concentration values used in training the prediction model to be described may be selected by choice. For example, in the case in which the hormone concentrations corresponding to the determined reference date include the T4 concentration, the free T4 concentration, the T3 concentration, the free T3 concentration, the TSH concentration, and the TRH concentration, only the free T3 and TSH concentrations may be obtained when only the free T4 and TSH concentrations are used in training the prediction model.

When a plurality of reference dates are determined, a hormone concentration corresponding to each of the plurality of reference dates may be obtained.

### Obtaining of the pre-processing result for the interval heart rates corresponding to the reference date in step S140

When pre-processing of the interval heart rates corresponding to the reference date is already performed and the pre-processing result is stored, the stored pre-processing result may be obtained.

When pre-processing of the interval heart rates corresponding to the reference date is not performed yet, pre-processing of the interval heart rates corresponding to the reference date may be performed.

The obtaining and pre-processing of the interval heart rates corresponding to the reference date is similar to the above-described obtaining and pre-processing of the interval heart rates corresponding to the target date, so a detailed description will be omitted.

Consequently, the pre-processing result for the interval heart rates with respect to the reference date may include at least one of the following: an average of the interval heart rates corresponding to the reference date, and a parameter related to the distribution of the interval heart rates corresponding to the reference date. Herein, the parameter related to the distribution of the interval heart rates corresponding to the reference date may include at least one of the following: a standard deviation of the interval heart rates corresponding to the target date, skewness of the interval heart rates corresponding to the target date, and kurtosis of the interval heart rates corresponding to the target date.

When a plurality of reference dates are determined, a pre-processing result for interval heart rates corresponding to each of the plurality of reference dates may be obtained.

In the meantime, among various pre-processing results of the interval heart rates corresponding to the reference date, only the pre-processing results related to the input values used in training the prediction model to be described later may be obtained. For example, when a rate of change in an average value and an amount of change in a relative standard deviation of interval heart rates with respect to a target date and a reference date are used in training the prediction model, only the average and standard deviation of the interval heart rates corresponding to the reference date may be obtained from the interval heart rates corresponding to the reference date. However, all or some of the above-described pre-processing results may be obtained regardless of the input values used in training the prediction model.

### Obtaining of the difference between the pre-processing result for the interval heart rates on the target date and the pre-processing result for the interval heart rates on the reference date in step S150

The difference between the pre-processing result for the interval heart rates on the target date and the pre-processing result for the interval heart rates on the reference date may be one of the following or a combination thereof.

1) an amount of change in the average of the interval heart rates corresponding to the target date (the period average heart rate on the target date) with respect to the average of the interval heart rates corresponding to the reference date (the period average heart rate on the reference date) (the period average heart rate on the target date - the period average heart rate on the reference date)
2) a rate of change in the average (of the interval heart rates corresponding to the target date the period average heart rate on the target date) with respect to the average of the interval heart rates corresponding to the reference date (the period average heart rate on the reference date) ((the period average heart rate on the target date - the period average heart rate on the reference date) / the period average heart rate on the reference date)
3) an amount of change in the standard deviation of the interval heart rates corresponding to the target date with respect to the standard deviation of the interval heart rates corresponding to the reference date (the standard deviation of the interval heart rates corresponding to the target date - the standard deviation of the interval heart rates corresponding to the reference date)
4) an amount of change in the relative standard deviation of the interval heart rates corresponding to the target date with respect to the relative standard deviation of the interval heart rates corresponding to the reference date ((the standard deviation of the interval heart rates corresponding to the target date / the period average heart rate on the target date) - (the standard deviation of the interval heart rates corresponding to the reference date / the period average heart rate on the reference date))
5) an amount of change in the skewness of the interval heart rates corresponding to the target date with respect to the skewness of the interval heart rates corresponding to the reference date (the skewness of the interval heart rates corresponding to the target date - the skewness of the interval heart rates corresponding to the reference date)
6) an amount of change in the kurtosis of the interval heart rates corresponding to the target date with respect to the kurtosis of the interval heart rates corresponding to the reference date (the kurtosis of the interval heart rates corresponding to the target date - the kurtosis of the interval heart rates corresponding to the reference date)
7) a JS divergence between the distribution of the interval heart rates corresponding to the target date and the distribution of the interval heart rates corresponding to the reference date

When a plurality of reference dates are determined, differences between pre-processing results for each of the plurality of reference dates may be obtained. For example, obtained may be a difference between first pre-processing results determined between a first reference date and a target date, and a difference between second pre-processing results determined between a second reference date and the target date.

In the meantime, among the differences between the pre-processing results, only the differences between the pre-processing results related to the input values used in training the prediction model to be described later may be obtained. For example, when a rate of change in an average value and an amount of change in a relative standard deviation of interval heart rates with respect to a target date and a reference date are used in training the prediction model, obtained are only the rate of change in the average (the period average heart rate on the target date) of the interval heart rates corresponding to the target date with respect to the average (the period average heart rate on the reference date) of the interval heart rates corresponding to the reference date, and the amount of change in the standard deviation of the interval heart rates corresponding to the target date with respect to the standard deviation of the interval heart rates corresponding to the reference date. However, all or some of the above-described differences between the pre-processing results may be obtained regardless of the input values used in training the prediction model.

### Calculation of Combination Value

According to several embodiments described in the present application, a combination value of variables may be calculated.

When a first period average heart rate, a first standard deviation, a first skewness, a first kurtosis, a first TSH hormone concentration, a first free T4 concentration, a first T4 concentration, a first free T3 concentration, a first T3 concentration, a first TRH concentration, and a diagnosis result for thyroid dysfunction with respect to a first reference date correspond to the first reference date, and a second period average heart rate, a second standard deviation, a second skewness, a second kurtosis, a second TSH hormone concentration, a second free T4 concentration, a second T4 concentration, a second free T3 concentration, a second T3 concentration, a second TRH concentration, and a diagnosis result for thyroid dysfunction with respect to a second reference date correspond to the second reference date, the variables are calculated as follows.
(1) An amount of change in a period average heart rate = the period average heart rate on the second reference date - the period average heart rate on the first reference date,
(2) A rate of change in a period average heart rate = (the period average heart rate on the second reference date - the period average heart rate on the first reference date) / the period average heart rate on the second reference date,
(3) An amount of change in a standard deviation = the standard deviation of interval heart rates on the second reference date - the standard deviation of interval heart rates on the first reference date,
(4) An amount of change in a relative standard deviation = (the standard deviation of interval heart rates on the second reference date / the period average heart rate on the second reference date) - (the standard deviation of interval heart rates on the first reference date / the period average heart rate on the first reference date),
(5) An amount of change in skewness of interval heart rates = the skewness of interval heart rates on the second reference date - the skewness of interval heart rates on the first reference date,
(6) An amount of change in kurtosis of interval heart rates = the kurtosis of interval heart rates on the second reference date - the skewness of interval heart rates on the first reference date,
(7) JS divergence : a JS divergence calculated between interval heart rates on the second reference date and interval heart rates on the first reference date,
(8) First TSH hormone concentration,
(9) First free T4 concentration,
(10) First T4 concentration,
(11) First free T3 concentration,
(12) First T3 concentration,
(13) First TRH concentration,
(14) Second TSH hormone concentration,
(15) Second free T4 concentration,
(16) Second T4 concentration,
(17) Second free T3 concentration,
(18) Second T3 concentration, and
(19) Second TRH concentration

Herein, a combination value of the variables means one of the following: a value obtained by multiplying one value (hereinafter, referred to as a first value) selected among the variables, by another value (hereinafter, referred to as a second value) selected among the variables; a value obtained by dividing the first value by the second value; and a value obtained by dividing the second value by the first value. For example, the combination value may be a value obtained by dividing a free T4 concentration value on the second reference date by a JS divergence.

### Calculation of Day Gap between Target Date and Reference Date

According to several embodiments described in the present application, a day gap between the target date and the reference date may be obtained.

### Acquisition of Personal Information of User

According to several embodiments described in the present application, personal information of a subject, that is, personal information of a user, may be obtained.

### The obtaining of the prediction result for thyroid dysfunction through the thyroid dysfunction prediction model in step S160

### Acquisition of Prediction Result for Hyperthyroidism through Hyperthyroidism Prediction Model

To the hyperthyroidism prediction model trained as described in Training Method Embodiment #1, the following may be input: the obtained hormone concentration on the reference date, and the obtained amount of change in the average of the interval heart rates corresponding to the target date (the period average heart rate on the target date) with respect to the average (of the interval heart rates corresponding to the reference date the period average heart rate on the reference date). Accordingly, a result on whether the subject has or does not have hyperthyroidism may be obtained. That is, a prediction result for hyperthyroidism may be obtained.

To the hyperthyroidism prediction model trained as described in Training Method Embodiment #2, the following may be input: the obtained hormone concentration on the reference date, the obtained amount of change in the average of the interval heart rates corresponding to the target date (the period average heart rate on the target date) with respect to the average of the interval heart rates corresponding to the reference date (the period average heart rate on the reference date), and the period average heart rate corresponding to the target date. Accordingly, a result on whether the subject has or does not have hyperthyroidism may be obtained. That is, a prediction result for hyperthyroidism may be obtained.

To the hyperthyroidism prediction model trained as described in Training Method Embodiment #3, the following may be input: the obtained hormone concentration on the reference date, the obtained amount of change in the average of the interval heart rates corresponding to the target date (the period average heart rate on the target date) with respect to the average of the interval heart rates corresponding to the reference date (the period average heart rate on the reference date), and the difference between the parameter related to the distribution of the interval heart rates corresponding to the target date and the parameter related to the distribution of the interval heart rates corresponding to the reference date. Accordingly, a result on whether the subject has or does not have hyperthyroidism may be obtained. That is, a prediction result for hyperthyroidism may be obtained. Herein, the difference between the parameter related to the distribution of the interval heart rates corresponding to the target date and the parameter related to the distribution of the interval heart rates corresponding to the reference date may be one selected from a group consisting of the following or a combination thereof.
1) an amount of change in the standard deviation of the interval heart rates corresponding to the target date with respect to the standard deviation of the interval heart rates corresponding to the reference date (the standard deviation of the interval heart rates corresponding to the target date - the standard deviation of the interval heart rates corresponding to the reference date)
2) an amount of change in the skewness of the interval heart rates corresponding to the target date with respect to the skewness of the interval heart rates corresponding to the reference date (the skewness of the interval heart rates corresponding to the target date - the skewness of the interval heart rates corresponding to the reference date)
3) an amount of change in the kurtosis of the interval heart rates corresponding to the target date with respect to the kurtosis of the interval heart rates corresponding to the reference date (the kurtosis of the interval heart rates corresponding to the target date - the kurtosis of the interval heart rates corresponding to the reference date)
4) a JS divergence between the distribution of the interval heart rates corresponding to the target date and the distribution of the interval heart rates corresponding to the reference date

To the hyperthyroidism prediction model trained as described in Training Method Embodiment #4, the following may be input: the obtained hormone concentration on the reference date, the obtained amount of change in the average of the interval heart rates corresponding to the target date (the period average heart rate on the target date) with respect to the average of the interval heart rates corresponding to the reference date (the period average heart rate on the reference date), the average of the interval heart rates corresponding to the target date, and the difference between the parameter related to the distribution of the interval heart rates corresponding to the target date and the parameter related to the distribution of the interval heart rates corresponding to the reference date. Accordingly, a result on whether the subject has or does not have hyperthyroidism may be obtained. That is, a prediction result for hyperthyroidism may be obtained.

To the hyperthyroidism prediction model trained as described in Training Method Embodiment #5, the following may be input: the obtained hormone concentration on the reference date, and the obtained rate of change in the average of the interval heart rates corresponding to the target date (the period average heart rate on the target date) with respect to the average of the interval heart rates corresponding to the reference date (the period average heart rate on the reference date). Accordingly, a result on whether the subject has or does not have hyperthyroidism may be obtained. That is, a prediction result for hyperthyroidism may be obtained.

To the hyperthyroidism prediction model trained as described in Training Method Embodiment #6, the following may be input: the obtained hormone concentration on the reference date, the obtained rate of change in the average of the interval heart rates corresponding to the target date (the period average heart rate on the target date) with respect to the average of the interval heart rates corresponding to the reference date (the period average heart rate on the reference date), and the average of the interval heart rates corresponding to the target date. Accordingly, a result on whether the subject has or does not have hyperthyroidism may be obtained. That is, a prediction result for hyperthyroidism may be obtained.

To the hyperthyroidism prediction model trained as described in Training Method Embodiment #7, the following may be input: the obtained hormone concentration on the reference date, the obtained rate of change in the average of the interval heart rates corresponding to the target date (the period average heart rate on the target date) with respect to the average of the interval heart rates corresponding to the reference date (the period average heart rate on the reference date), and the difference between the parameter related to the distribution of the interval heart rates corresponding to the target date and the parameter related to the distribution of the interval heart rates corresponding to the reference date. Accordingly, a result on whether the subject has or does not have hyperthyroidism may be obtained. That is, a prediction result for hyperthyroidism may be obtained. Herein, the difference between the parameter related to the distribution of the interval heart rates corresponding to the target date and the parameter related to the distribution of the interval heart rates corresponding to the reference date may be one selected from a group consisting of the following or a combination thereof.
1) an amount of change in the relative standard deviation of the interval heart rates corresponding to the target date with respect to the relative standard deviation of the interval heart rates corresponding to the reference date ((the standard deviation of the interval heart rates corresponding to the target date / the period average heart rate on the target date) - (the standard deviation of the interval heart rates corresponding to the reference date / the period average heart rate on the reference date))
2) an amount of change in the skewness of the interval heart rates corresponding to the target date with respect to the skewness of the interval heart rates corresponding to the reference date (the skewness of the interval heart rates corresponding to the target date - the skewness of the interval heart rates corresponding to the reference date)
3) an amount of change in the kurtosis of the interval heart rates corresponding to the target date with respect to the kurtosis of the interval heart rates corresponding to the reference date (the kurtosis of the interval heart rates corresponding to the target date - the kurtosis of the interval heart rates corresponding to the reference date)
4) a JS divergence between the distribution of the interval heart rates corresponding to the target date and the distribution of the interval heart rates corresponding to the reference date

To the hyperthyroidism prediction model trained as described in Training Method Embodiment #8, the following may be input: the obtained hormone concentration on the reference date, the obtained rate of change in the average of the interval heart rates corresponding to the target date (the period average heart rate on the target date) with respect to the average of the interval heart rates corresponding to the reference date (the period average heart rate on the reference date), the average of the interval heart rates corresponding to the target date, and the difference between the parameter related to the distribution of the interval heart rates corresponding to the target date and the parameter related to the distribution of the interval heart rates corresponding to the reference date. Accordingly, a result on whether the subject has or does not have hyperthyroidism may be obtained. That is, a prediction result for hyperthyroidism may be obtained. Herein, the difference between the parameter related to the distribution of the interval heart rates corresponding to the target date and the parameter related to the distribution of the interval heart rates corresponding to the reference date may be one selected from a group consisting of the following or a combination thereof.
1) an amount of change in the relative standard deviation of the interval heart rates corresponding to the target date with respect to the relative standard deviation of the interval heart rates corresponding to the reference date ((the standard deviation of the interval heart rates corresponding to the target date / the period average heart rate on the target date) - (the standard deviation of the interval heart rates corresponding to the reference date / the period average heart rate on the reference date))
2) an amount of change in the skewness of the interval heart rates corresponding to the target date with respect to the skewness of the interval heart rates corresponding to the reference date (the skewness of the interval heart rates corresponding to the target date - the skewness of the interval heart rates corresponding to the reference date)
3) an amount of change in the kurtosis of the interval heart rates corresponding to the target date with respect to the kurtosis of the interval heart rates corresponding to the reference date (the kurtosis of the interval heart rates corresponding to the target date - the kurtosis of the interval heart rates corresponding to the reference date)
4) a JS divergence between the distribution of the interval heart rates corresponding to the target date and the distribution of the interval heart rates corresponding to the reference date

To the hyperthyroidism prediction model trained as described in Training Method Embodiment #9, the following may be input: the obtained hormone concentration on the reference date, the obtained rate of change (or amount of change) in the average of the interval heart rates corresponding to the target date (the period average heart rate on the target date) with respect to the average of the interval heart rates corresponding to the reference date (the period average heart rate on the reference date), the difference between the parameter related to the distribution of the interval heart rates corresponding to the target date and the parameter related to the distribution of the interval heart rates corresponding to the reference date, and the day gap between the target date and the reference date. Accordingly, a result on whether the subject has or does not have hyperthyroidism may be obtained. That is, a prediction result for hyperthyroidism may be obtained. Herein, the difference between the parameter related to the distribution of the interval heart rates corresponding to the target date and the parameter related to the distribution of the interval heart rates corresponding to the reference date may be one selected from a group consisting of the following or a combination thereof.
1) an amount of change in the standard deviation of the interval heart rates corresponding to the target date with respect to the standard deviation of the interval heart rates corresponding to the reference date (the standard deviation of the interval heart rates corresponding to the target date - the standard deviation of the interval heart rates corresponding to the reference date)
2) an amount of change in the relative standard deviation of the interval heart rates corresponding to the target date with respect to the relative standard deviation of the interval heart rates corresponding to the reference date ((the standard deviation of the interval heart rates corresponding to the target date / the period average heart rate on the target date) - (the standard deviation of the interval heart rates corresponding to the reference date / the period average heart rate on the reference date))
3) an amount of change in the skewness of the interval heart rates corresponding to the target date with respect to the skewness of the interval heart rates corresponding to the reference date (the skewness of the interval heart rates corresponding to the target date - the skewness of the interval heart rates corresponding to the reference date)
4) an amount of change in the kurtosis of the interval heart rates corresponding to the target date with respect to the kurtosis of the interval heart rates corresponding to the reference date (the kurtosis of the interval heart rates corresponding to the target date - the kurtosis of the interval heart rates corresponding to the reference date)
5) a JS divergence between the distribution of the interval heart rates corresponding to the target date and the distribution of the interval heart rates corresponding to the reference date

To the hyperthyroidism prediction model trained as described in Training Method Embodiment #10, the following may be input: the obtained hormone concentration on the reference date, the obtained rate of change (or amount of change) in the average of the interval heart rates corresponding to the target date (the period average heart rate on the target date) with respect to the average of the interval heart rates corresponding to the reference date (the period average heart rate on the reference date), the difference between the parameter related to the distribution of the interval heart rates corresponding to the target date and the parameter related to the distribution of the interval heart rates corresponding to the reference date, and a combination value of variables. Accordingly, a result on whether the subject has or does not have hyperthyroidism may be obtained. That is, a prediction result for hyperthyroidism may be obtained.

Herein, the difference between the parameter related to the distribution of the interval heart rates corresponding to the target date and the parameter related to the distribution of the interval heart rates corresponding to the reference date may be one selected from a group consisting of the following or a combination thereof.
1) an amount of change in the standard deviation of the interval heart rates corresponding to the target date with respect to the standard deviation of the interval heart rates corresponding to the reference date (the standard deviation of the interval heart rates corresponding to the target date - the standard deviation of the interval heart rates corresponding to the reference date)
2) an amount of change in the relative standard deviation of the interval heart rates corresponding to the target date with respect to the relative standard deviation of the interval heart rates corresponding to the reference date ((the standard deviation of the interval heart rates corresponding to the target date / the period average heart rate on the target date) - (the standard deviation of the interval heart rates corresponding to the reference date / the period average heart rate on the reference date))
3) an amount of change in the skewness of the interval heart rates corresponding to the target date with respect to the skewness of the interval heart rates corresponding to the reference date (the skewness of the interval heart rates corresponding to the target date - the skewness of the interval heart rates corresponding to the reference date)
4) an amount of change in the kurtosis of the interval heart rates corresponding to the target date with respect to the kurtosis of the interval heart rates corresponding to the reference date (the kurtosis of the interval heart rates corresponding to the target date - the kurtosis of the interval heart rates corresponding to the reference date)
5) a JS divergence between the distribution of the interval heart rates corresponding to the target date and the distribution of the interval heart rates corresponding to the reference date

In the meantime, the combination value of variables has been described in detail in Training Method Embodiment #10, so a detailed description will be omitted.

To the hyperthyroidism prediction model trained as described in Training Method Embodiment #11, the following values may be input. Accordingly, a result on whether the subject has or does not have hyperthyroidism may be obtained. That is, a prediction result for hyperthyroidism may be obtained.
(1) the obtained hormone concentration on the reference date, the obtained amount of change in the average of the interval heart rates corresponding to the target date (the period average heart rate on the target date) with respect to the average of the interval heart rates corresponding to the reference date (the period average heart rate on the reference date), and the subject's age and/or sex
(2) the obtained hormone concentration on the reference date, the obtained amount of change in the average of the interval heart rates corresponding to the target date (the period average heart rate on the target date) with respect to the average of the interval heart rates corresponding to the reference date (the period average heart rate on the reference date), the period average heart rate corresponding to the target date, and the subject's age and/or sex
(3) the obtained hormone concentration on the reference date, the obtained amount of change in the average of the interval heart rates corresponding to the target date (the period average heart rate on the target date) with respect to the average of the interval heart rates corresponding to the reference date (the period average heart rate on the reference date), the difference between the parameter related to the distribution of the interval heart rates corresponding to the target date and the parameter related to the distribution of the interval heart rates corresponding to the reference date, and the subject's age and/or sex
(4) the obtained hormone concentration on the reference date, the obtained amount of change in the average of the interval heart rates corresponding to the target date (the period average heart rate on the target date) with respect to the average of the interval heart rates corresponding to the reference date (the period average heart rate on the reference date), the average of the interval heart rates corresponding to the target date, the difference between the parameter related to the distribution of the interval heart rates corresponding to the target date and the parameter related to the distribution of the interval heart rates corresponding to the reference date, and the subject's age and/or sex
(5) the obtained hormone concentration on the reference date, the obtained rate of change in the average of the interval heart rates corresponding to the target date (the period average heart rate on the target date) with respect to the average of the interval heart rates corresponding to the reference date (the period average heart rate on the reference date), and the subject's age and/or sex
(6) the obtained hormone concentration on the reference date, the obtained rate of change in the average of the interval heart rates corresponding to the target date (the period average heart rate on the target date) with respect to the average of the interval heart rates corresponding to the reference date (the period average heart rate on the reference date), the average of the interval heart rates corresponding to the target date, and the subject's age and/or sex
(7) the obtained hormone concentration on the reference date, the obtained rate of change in the average of the interval heart rates corresponding to the target date (the period average heart rate on the target date) with respect to the average of the interval heart rates corresponding to the reference date (the period average heart rate on the reference date), the difference between the parameter related to the distribution of the interval heart rates corresponding to the target date and the parameter related to the distribution of the interval heart rates corresponding to the reference date, and the subject's age and/or sex
(8) the obtained hormone concentration on the reference date, the obtained rate of change in the average of the interval heart rates corresponding to the target date (the period average heart rate on the target date) with respect to the average of the interval heart rates corresponding to the reference date (the period average heart rate on the reference date), the average of the interval heart rates corresponding to the target date, the difference between the parameter related to the distribution of the interval heart rates corresponding to the target date and the parameter related to the distribution of the interval heart rates corresponding to the reference date, and the subject's age and/or sex
(9) the obtained hormone concentration on the reference date, the obtained rate of change (or amount of change) in the average of the interval heart rates corresponding to the target date (the period average heart rate on the target date) with respect to the average of the interval heart rates corresponding to the reference date (the period average heart rate on the reference date), the difference between the parameter related to the distribution of the interval heart rates corresponding to the target date and the parameter related to the distribution of the interval heart rates corresponding to the reference date, the day gap between the target date and the reference date, and the subject's age and/or sex
   (9) the obtained hormone concentration on the reference date, the obtained rate of change (or amount of change) in the average of the interval heart rates corresponding to the target date (the period average heart rate on the target date) with respect to the average of the interval heart rates corresponding to the reference date (the period average heart rate on the reference date), the difference between the parameter related to the distribution of the interval heart rates corresponding to the target date and the parameter related to the distribution of the interval heart rates corresponding to the reference date, a combination value of variables, and the subject's age and/or sex

### Acquisition of Prediction Result for Hypothyroidism through Hypothyroidism Prediction Model

Similarly to the description of obtaining the prediction result for hyperthyroidism through the hyperthyroidism prediction model, a prediction result for hypothyroidism may be obtained through the hypothyroidism prediction model. Therefore, a detailed description will be omitted.

### Acquisition of Prediction Result for Thyroid Dysfunction

The prediction result for hyperthyroidism and the prediction result for hypothyroidism are synthesized, thereby obtaining a prediction result for thyroid dysfunction for the subject.

A prediction result for thyroid dysfunction considering a prediction result A for hyperthyroidism and a prediction result B for hypothyroidism may be obtained as shown in [Table 2] below.

**[Table 2]**

| A | B | Thyroid dysfunction prediction results |
|---|---|---|
| Hyperthyroidism | Hypothyroidism | Hyperthyroidism or Hypothyroidism |
| Non-hyperthyroidism | Non-hypothyroidism | Normal |
| Hyperthyroidism | Non-hypothyroidism | Hyperthyroidism |
| Non-hyperthyroidism | Hypothyroidism | Hypothyroidism |

In the meantime, when the prediction result for hyperthyroidism is hyperthyroidism and the prediction result for hypothyroidism is hypothyroidism, an output value (probability value) of the hyperthyroidism prediction model is compared with an output value (probability value) of the hypothyroidism prediction model and the result with the output of the higher probability value may be adopted as the final result for thyroid dysfunction.

The heart rate measuring device 10 may perform all the above-described steps including: obtaining a trigger signal in step S100; obtaining interval heart rates corresponding to a target date determined by the trigger signal, in step S110; preprocessing the interval heart rates corresponding to the target date in step S120; obtaining a hormone concentration corresponding to a reference date in step S130; obtaining a pre-processing result for interval heart rates corresponding to the reference date in step S140; obtaining a difference between a pre-processing result for the interval heart rates corresponding to the target date and the pre-processing result for the interval heart rates corresponding to the reference date in step S150; and processing the obtained difference and the hormone concentration corresponding to the reference date by using the thyroid dysfunction prediction model, and obtaining a result for thyroid dysfunction for the subject, in step S160.

The user terminal 20 may perform all the above-described steps including: obtaining a trigger signal in step S100; obtaining interval heart rates corresponding to a target date determined by the trigger signal, in step S110; preprocessing the interval heart rates corresponding to the target date in step S120; obtaining a hormone concentration corresponding to a reference date in step S130; obtaining a pre-processing result for interval heart rates corresponding to the reference date in step S140; obtaining a difference between a pre-processing result for the interval heart rates corresponding to the target date and the pre-processing result for the interval heart rates corresponding to the reference date in step S150; and processing the obtained difference and the hormone concentration corresponding to the reference date by using the thyroid dysfunction prediction model, and obtaining a result for thyroid dysfunction for the subject, in step S160.

The server 30 may perform all the above-described steps including: obtaining a trigger signal in step S100; obtaining interval heart rates corresponding to a target date determined by the trigger signal, in step S110; preprocessing the interval heart rates corresponding to the target date in step S120; obtaining a hormone concentration corresponding to a reference date in step S130; obtaining a pre-processing result for interval heart rates corresponding to the reference date in step S140; obtaining a difference between a pre-processing result for the interval heart rates corresponding to the target date and the pre-processing result for the interval heart rates corresponding to the reference date in step S150; and processing the obtained difference and the hormone concentration corresponding to the reference date by using the thyroid dysfunction prediction model, and obtaining a result for thyroid dysfunction for the subject, in step S160.

The heart rate measuring device 10, the user terminal 20, and the server 30 may, in an appropriately distributed manner, perform the above-described steps including: obtaining a trigger signal in step S100; obtaining interval heart rates corresponding to a target date determined by the trigger signal, in step S110; preprocessing the interval heart rates corresponding to the target date in step S120; obtaining a hormone concentration corresponding to a reference date in step S130; obtaining a pre-processing result for interval heart rates corresponding to the reference date in step S140; obtaining a difference between a pre-processing result for the interval heart rates corresponding to the target date and the pre-processing result for the interval heart rates corresponding to the reference date in step S150; and processing the obtained difference and the hormone concentration corresponding to the reference date by using the thyroid dysfunction prediction model, and obtaining a result for thyroid dysfunction for the subject, in step S160.

For example, the user terminal 20 may perform the following steps: obtaining a trigger signal in step S100; obtaining interval heart rates corresponding to a target date determined by the trigger signal, in step S110; preprocessing the interval heart rates corresponding to the target date in step S120. The server 30 may perform the following steps: obtaining a hormone concentration corresponding to a reference date in step S130; obtaining a pre-processing result for interval heart rates corresponding to the reference date in step S140; obtaining a difference between a pre-processing result for the interval heart rates corresponding to the target date and the pre-processing result for the interval heart rates corresponding to the reference date in step S150; and processing the obtained difference and the hormone concentration corresponding to the reference date by using the thyroid dysfunction prediction model, and obtaining a result for thyroid dysfunction for the subject, in step S160.

As another example, the user terminal 20 may perform obtaining a trigger signal in step S100. The server 30 may perform the following steps: obtaining interval heart rates corresponding to a target date determined by the trigger signal, in step S110; preprocessing the interval heart rates corresponding to the target date in step S120; obtaining a hormone concentration corresponding to a reference date in step S130; obtaining a pre-processing result for interval heart rates corresponding to the reference date in step S140; obtaining a difference between a pre-processing result for the interval heart rates corresponding to the target date and the pre-processing result for the interval heart rates corresponding to the reference date in step S150; and processing the obtained difference and the hormone concentration corresponding to the reference date by using the thyroid dysfunction prediction model, and obtaining a result for thyroid dysfunction for the subject, in step S160.

However, the form of the above-described distributed performance is not limited thereto, and distributed performance may be achieved in various forms.

### 5. Experimental Examples

Hereinafter, described will be experimental examples in which the thyroid dysfunction prediction model described in the present application was trained, and the results of analyzing accuracy.

The clinical data used in the experimental examples and the comparative examples described below were collected by the above-described method, and a total of 1,027 clinical data sets collected from a total of 297 patients were used. Among these, a total of 168 clinical data sets were classified as having a diagnosis result "hyperthyroidism" for hormone concentration, a total of 801 clinical data sets were classified as having a diagnosis result "normal", and a total of 58 clinical data sets were classified as having a diagnosis result "hypothyroidism".

### (1) Comparative Example #1 - Heart Rate Difference with respect to Reference Date for Normal - Labelling

### Preparation of Training Data Sets

In order to train the prediction model according to the comparative example for comparison with experimental results of various embodiments described in the present application, training data sets were prepared in the following way.

For each of the patients, a value obtained by subtracting a period average heart rate corresponding to a test date with a diagnosis result "normal" from a period average heart rate corresponding to a test date with a diagnosis result "abnormal" was used as an input value (for example, an amount of change in a period average heart rate), and a diagnosis result (hyperthyroidism or hypothyroidism) corresponding to the test date with the diagnosis result "abnormal" was used as a labeling value. In addition, when there were two or more test dates with a diagnosis result "normal", a value obtained by subtracting a period average heart rate corresponding to one test date (a test date with a diagnosis result "normal") from a period average heart rate corresponding to another test date (another test date with a diagnosis result "normal") was used as an input value, and non-hyperthyroidism or non-hypothyroidism was used as a labeling value.

Herein, regarding the period average heart rate corresponding to the test date, resting heart rates for 10 days were obtained on the basis of the test date, and an average of the obtained resting heart rates was calculated.

Herein, the training data sets were not generated using a "difference" in clinical data between different patients, but the training data sets were generated using clinical data sets obtained from one patient.

Herein, as the clinical data used for training, 495 pieces of data obtained from 157 patients were used, and a total of 1,199 training data sets were generated therefrom.

### Training of Hyperthyroidism Prediction Model

Using the light gradient boosting machine, the hyperthyroidism prediction model was trained with the above-described training data sets.

### Test of Hyperthyroidism Prediction Model

The test data sets used to determine the accuracy of the trained prediction model were generated from 437 pieces of clinical data obtained from a total of 121 patients, and a total of 1,138 test data sets were generated.

The accuracy, sensitivity, specificity, positive predictive value (PPV), and negative predictive value (NPV) of the hyperthyroidism prediction model according to Comparative Example #1 were as shown in [Table 3] below.

**[Table 3]**

| | Accuracy | Sensitivity | Specificity | PPV | NPV |
|---|---|---|---|---|---|
| Comparative Example #1 | 71.62 | 60.66 | 73.43 | 27.01 | 92.00 |

### (2) Experimental Example #1 - Heart Rate Difference + Hormone Level (VS Comparative Example #1)

### Preparation of Training Data Sets

A total of 563 clinical data sets collected from a total of 171 patients were used.

According to the method of training the hyperthyroidism prediction model described above in Training Method Embodiment #1, the model was trained. That is, generated were training data sets in the format such as [(a period average heart rate corresponding to a first test date - a period average heart rate corresponding to a second test date), a thyroid hormone concentration corresponding to the second test date, a diagnosis result (hyperthyroidism or non-hyperthyroidism) corresponding to the first test date], and the training data sets were used in training the model.

Herein, regarding the period average heart rate corresponding to the test date, resting heart rates for 10 days were obtained on the basis of the test date, and an average of the obtained resting heart rates was calculated.

In addition, regarding the used hormone level, a method using only a free T4 concentration and a method using both a free T4 and a TSH concentration were used together.

Herein, the training data sets were not generated using a "difference" in clinical data between different patients, but the training data sets were generated using clinical data sets obtained from one patient.

Herein, as the clinical data used for training, 563 pieces of data secured from 171 patients were used, and a total of 1,542 training data sets were generated therefrom.

### Training of Hyperthyroidism Prediction Model

Using the light gradient boosting machine, the hyperthyroidism prediction model was trained with the above-described training data sets.

### Test of Hyperthyroidism Prediction Model

The test data sets used to determine the accuracy of the trained prediction model were generated from 464 pieces of clinical data secured from a total of 126 patients, and a total of 1,416 test data sets were generated.

According to Experimental Example #1, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #1 were as shown in [Table 4] below.

**[Table 4]**

| | Accuracy | Sensitivity | Specificity | PPV | NPV |
|---|---|---|---|---|---|
| Free T4 | 82.33 | 78.38 | 83.08 | 46.77 | 95.29 |
| Free T4 + TSH | 82.54 | 86.49 | 81.79 | 47.41 | 96.96 |

The inventors of the present application found that it is sufficiently possible to predict whether there is "hyperthyroidism" by determining a reference data regardless of whether there is "normal" of "hyperthyroidism" and by using a difference in a period average heart rate between a reference data and an analysis target date with a hormone concentration corresponding to the reference date without determining a date when the hormone level is "normal" as a reference date and without using a difference in a period average heart rate between the reference date and an analysis target date like comparative Example #1, and found that results such as accuracy and sensitivity are rather much greater.

Accordingly, more training data sets are obtained from the same level of clinical data, and the prediction model capable of predicting thyroid dysfunction more accurately is obtained.

### (3) Experimental Example #2 - Heart Rate Difference + Current Heart Rate + Hormone Level

### Preparation of Training Data Sets

A total of 563 clinical data sets collected from a total of 171 patients were used.

According to the method of training the hyperthyroidism prediction model described above in Training Method Embodiment #2, the model was trained. That is, generated were training data sets in the format such as [(a period average heart rate corresponding to a first test date - a period average heart rate corresponding to a second test date), the period average heart rate corresponding to the first test date, a thyroid hormone concentration corresponding to the second test date, a diagnosis result (hyperthyroidism or non-hyperthyroidism) corresponding to the first test date], and the training data sets were used in training the model.

Herein, regarding the period average heart rate corresponding to the test date, resting heart rates for 10 days were obtained on the basis of the test date, and an average of the obtained resting heart rates was calculated.

In addition, regarding the used hormone level, a method using only a free T4 concentration and a method using both a free T4 and a TSH concentration were used together.

Herein, the training data sets were not generated using a "difference" in clinical data between different patients, but the training data sets were generated using clinical data sets obtained from one patient.

Herein, as the clinical data used for training, 563 pieces of data secured from 171 patients were used, and a total of 1,542 training data sets were generated therefrom.

### Training of Hyperthyroidism Prediction Model

Using the light gradient boosting machine, the hyperthyroidism prediction model was trained with the above-described training data sets.

### Test of Hyperthyroidism Prediction Model

The test data sets used to determine the accuracy of the trained prediction model were generated from 464 pieces of clinical data secured from a total of 126 patients, and a total of 1,416 test data sets were generated.

According to Experimental Example #2, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #2 were as shown in [Table 5] below.

**[Table 5]**

| | Accuracy | Sensitivity | Specificity | PPV | NPV |
|---|---|---|---|---|---|
| Free T4 | 83.62 | 71.62 | 85.90 | 49.07 | 94.10 |
| Free T4 + TSH | 85.34 | 74.32 | 87.44 | 52.88 | 94.72 |

The inventors of the present application found that in predicting hyperthyroidism, accuracy is slightly increased when an amount of change in a period average heart rate and a period average heart rate (current period average heart rate) on a target date are used as input values.

### (4) Experimental Example #3 - Heart Rate Difference + Hormone Level + Standard Deviation

### Preparation of Training Data Sets

A total of 563 clinical data sets collected from a total of 171 patients were used.

According to the method of training the hyperthyroidism prediction model described above in Training Method Embodiment #3, the model was trained. That is, generated were training data sets in the format such as [(a period average heart rate corresponding to a first test date - a period average heart rate corresponding to a second test date), (a standard deviation of interval heart rates corresponding to the first test date - a standard deviation of interval heart rates corresponding to the second test date), a thyroid hormone concentration corresponding to the second test date, a diagnosis result (hyperthyroidism or non-hyperthyroidism) corresponding to the first test date], and the training data sets were used in training the model.

Herein, regarding the period average heart rate corresponding to the test date, resting heart rates for 10 days were obtained on the basis of the test date, and an average of the obtained resting heart rates was calculated.

In addition, regarding the used hormone level, both a free T4 and a TSH concentration were used as input values.

Herein, the training data sets were not generated using a "difference" in clinical data between different patients, but the training data sets were generated using clinical data sets obtained from one patient.

Herein, as the clinical data used for training, 563 pieces of data secured from 171 patients were used, and a total of 1,542 training data sets were generated therefrom.

### Training of Hyperthyroidism Prediction Model

Using the light gradient boosting machine, the hyperthyroidism prediction model was trained with the above-described training data sets.

### Test of Hyperthyroidism Prediction Model

The test data sets used to determine the accuracy of the trained prediction model were generated from 464 pieces of clinical data secured from a total of 126 patients, and a total of 1,416 test data sets were generated.

According to Experimental Example #3, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #3 were as shown in [Table 6] below.

**[Table 6]**

| | Accuracy | Sensitivity | Specificity | PPV | NPV |
|---|---|---|---|---|---|
| Free T4 + TSH | 81.03 | 79.73 | 81.28 | 44.70 | 95.48 |

The inventors of the present application found that in predicting hyperthyroidism, accuracy is slightly increased when an amount of change in a period average heart rate and a period average heart rate (current period average heart rate) on a target date are used as input values.

### (5) Experimental Example #4 - Heart Rate Difference + Hormone Level + Standard Deviation Change + Skewness

### Preparation of Training Data Sets

A total of 563 clinical data sets collected from a total of 171 patients were used.

According to the method of training the hyperthyroidism prediction model described above in Training Method Embodiment #4, the model was trained. However, a standard deviation and skewness were used as parameters related to distribution. That is, generated were training data sets in the format such as [(a period average heart rate corresponding to a first test date - a period average heart rate corresponding to a second test date), (a standard deviation of interval heart rates corresponding to the first test date - a standard deviation of interval heart rates corresponding to the second test date), (the skewness of interval heart rates corresponding to the first test date - the skewness of interval heart rates corresponding to the second test date), a thyroid hormone concentration corresponding to the second test date, a diagnosis result (hyperthyroidism or non-hyperthyroidism) corresponding to the first test date], and the training data sets were used in training the model.

Herein, regarding the period average heart rate corresponding to the test date, resting heart rates for 10 days were obtained on the basis of the test date, and an average of the obtained resting heart rates was calculated.

In addition, regarding the used hormone level, both a free T4 and a TSH concentration were used as input values.

Herein, the training data sets were not generated using a "difference" in clinical data between different patients, but the training data sets were generated using clinical data sets obtained from one patient.

Herein, as the clinical data used for training, 563 pieces of data secured from 171 patients were used, and a total of 1,542 training data sets were generated therefrom.

### Training of Hyperthyroidism Prediction Model

Using the light gradient boosting machine, the hyperthyroidism prediction model was trained with the above-described training data sets.

### Test of Hyperthyroidism Prediction Model

The test data sets used to determine the accuracy of the trained prediction model were generated from 464 pieces of clinical data secured from a total of 126 patients, and a total of 1,416 test data sets were generated.

According to Experimental Example #4, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #4 were as shown in [Table 7] below.

**[Table 7]**

| | Accuracy | Sensitivity | Specificity | PPV | NPV |
|---|---|---|---|---|---|
| Free T4 + TSH | 83.62 | 81.08 | 84.10 | 49.18 | 95.91 |

### (6) Experimental Example #5 - Heart Rate Difference + Hormone Level + Standard Deviation Change + Kurtosis

### Preparation of Training Data Sets

Compared to Experimental Example #4, in Experimental Example #5, training data sets and test data sets were generated in the same way except that a standard deviation and kurtosis were used instead of using a standard deviation and skewness as parameters related to distribution.

### Training of Hyperthyroidism Prediction Model

Using the light gradient boosting machine, the hyperthyroidism prediction model was trained with the above-described training data sets.

### Test of Hyperthyroidism Prediction Model

According to Experimental Example #5, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #5 were as shown in [Table 8] below.

**[Table 8]**

| | Accuracy | Sensitivity | Specificity | PPV | NPV |
|---|---|---|---|---|---|
| Free T4 + TSH | 83.19 | 83.78 | 83.08 | 48.44 | 96.43 |

### (7) Experimental Example #6 - Heart Rate Difference + Hormone Level + Standard Deviation Change + JS divergence

### Preparation of Training Data Sets

Compared to Experimental Example #4, in Experimental Example #6, training data sets and test data sets were generated in the same way except that a JS divergence was used instead of using a difference in a standard deviation and a difference in skewness as differences in parameters related to distribution.

### Training of Hyperthyroidism Prediction Model

Using the light gradient boosting machine, the hyperthyroidism prediction model was trained with the above-described training data sets.

### Test of Hyperthyroidism Prediction Model

According to Experimental Example #6, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #6 were as shown in [Table 9] below.

**[Table 9]**

| | Accuracy | Sensitivity | Specificity | PPV | NPV |
|---|---|---|---|---|---|
| Free T4 + TSH | 83.19 | 78.38 | 84.10 | 48.33 | 95.35 |

As can be known through Experimental Examples #4, 5, and 6, the inventors of the present application found that in predicting hyperthyroidism, accuracy is slightly increased when an amount of change in a standard deviation as well as additional variables for observing a change in distribution of interval heart rates are used as input values.

### (8) Experimental Example #7 - Rate of Change in Heart Rates + Hormone Level + Relative Standard Deviation Change

### Preparation of Training Data Sets

A total of 563 clinical data sets collected from a total of 171 patients were used.

According to the method of training the hyperthyroidism prediction model described above in Training Method Embodiment #5, the model was trained. That is, generated were training data sets in the format such as [((a period average heart rate corresponding to a first test date - a period average heart rate corresponding to a second test date) / the period average heart rate corresponding to the second test date), (a relative standard deviation of interval heart rates corresponding to the first test date - a relative standard deviation of interval heart rates corresponding to the second test date), a thyroid hormone concentration corresponding to the second test date, a diagnosis result (hyperthyroidism or non-hyperthyroidism) corresponding to the first test date], and the training data sets were used in training the model.

Herein, regarding the period average heart rate corresponding to the test date, resting heart rates for 10 days were obtained on the basis of the test date, and an average of the obtained resting heart rates was calculated.

In addition, regarding the used hormone level, a method using both a free T4 and a TSH concentration was used.

Herein, the training data sets were not generated using a "difference" in clinical data between different patients, but the training data sets were generated using clinical data sets obtained from one patient.

Herein, as the clinical data used for training, 563 pieces of data secured from 171 patients were used, and a total of 1,542 training data sets were generated therefrom.

### Training of Hyperthyroidism Prediction Model

Using the light gradient boosting machine, the hyperthyroidism prediction model was trained with the above-described training data sets.

### Test of Hyperthyroidism Prediction Model

The test data sets used to determine the accuracy of the trained prediction model were generated from 464 pieces of clinical data secured from a total of 126 patients, and a total of 1,416 test data sets were generated.

According to Experimental Example #5, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #4 were as shown in [Table 10] below.

**[Table 10]**

| | Accuracy | Sensitivity | Specificity | PPV | NPV |
|---|---|---|---|---|---|
| Free T4 + TSH | 83.41 | 85.14 | 83.08 | 48.84 | 96.72 |

Compared to Experimental Example #1, Experimental Example #5 was an experiment conducted under the same conditions except that a rate of change in a period average heart rate and an amount of change in a relative standard deviation were used instead of using an amount of change in a period average heart rate and an amount of change in a standard deviation.

The inventors of the present application found that it is allowed to use a rate of change in a period average heart rate and an amount of change in a relative standard deviation as input values.

### (9) Experimental Example #8 - Rate of Change in Heart Rates + Hormone Level + Relative Standard Deviation Change + Day Gap

### Preparation of Training Data Sets

A total of 563 clinical data sets collected from a total of 171 patients were used.

According to the method of training the hyperthyroidism prediction model described above in Training Method Embodiment #9, the model was trained. That is, generated were training data sets in the format such as [((a period average heart rate corresponding to a first test date - a period average heart rate corresponding to a second test date) / the period average heart rate corresponding to the second test date), (a relative standard deviation of interval heart rates corresponding to the first test date - a relative standard deviation of interval heart rates corresponding to the second test date), a day gap between the second test date and the first test date, a thyroid hormone concentration corresponding to the second test date, a diagnosis result (hyperthyroidism or non-hyperthyroidism) corresponding to the first test date], and the training data sets were used in training the model.

Herein, regarding the period average heart rate corresponding to the test date, resting heart rates for 10 days were obtained on the basis of the test date, and an average of the obtained resting heart rates was calculated.

In addition, regarding the used hormone level, a method using both a free T4 and a TSH concentration was used.

Herein, the training data sets were not generated using a "difference" in clinical data between different patients, but the training data sets were generated using clinical data sets obtained from one patient.

Herein, as the clinical data used for training, 563 pieces of data secured from 171 patients were used, and a total of 1,542 training data sets were generated therefrom.

### Training of Hyperthyroidism Prediction Model

Using the light gradient boosting machine, the hyperthyroidism prediction model was trained with the above-described training data sets.

### Test of Hyperthyroidism Prediction Model

The test data sets used to determine the accuracy of the trained prediction model were generated from 464 pieces of clinical data secured from a total of 126 patients, and a total of 1,416 test data sets were generated.

According to Experimental Example #8, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #8 were as shown in [Table 11] below.

**[Table 11]**

| | Accuracy | Sensitivity | Specificity | PPV | NPV |
|---|---|---|---|---|---|
| Free T4 + TSH | 83.84 | 82.43 | 84.10 | 49.59 | 96.19 |

### (10) Experimental Example #9 - Rate of Change in Heart Rates + Hormone Level + Relative Standard Deviation Change + Combination Value

### Preparation of Training Data Sets

A total of 563 clinical data sets collected from a total of 171 patients were used.

According to the method of training the hyperthyroidism prediction model described above in Training Method Embodiment #10, the model was trained. That is, generated were training data sets in the format such as [((a period average heart rate corresponding to a first test date - a period average heart rate corresponding to a second test date) / the period average heart rate corresponding to the second test date), (a relative standard deviation of interval heart rates corresponding to the first test date - a relative standard deviation of interval heart rates corresponding to the second test date), a day gap between the second test date and the first test date, a thyroid hormone concentration corresponding to the second test date, a combination value of variables, a diagnosis result (hyperthyroidism or non-hyperthyroidism) corresponding to the first test date], and the training data sets were used in training the model.

Herein, regarding the period average heart rate corresponding to the test date, resting heart rates for 10 days were obtained on the basis of the test date, and an average of the obtained resting heart rates was calculated.

In addition, regarding the used hormone level, a method using both a free T4 and a TSH concentration was used.

Herein, the training data sets were not generated using a "difference" in clinical data between different patients, but the training data sets were generated using clinical data sets obtained from one patient.

Herein, as the clinical data used for training, 563 pieces of data secured from 171 patients were used, and a total of 1,542 training data sets were generated therefrom.

### Training of Hyperthyroidism Prediction Model

Using the light gradient boosting machine, the hyperthyroidism prediction model was trained with the above-described training data sets.

### Test of Hyperthyroidism Prediction Model

The test data sets used to determine the accuracy of the trained prediction model were generated from 464 pieces of clinical data secured from a total of 126 patients, and a total of 1,416 test data sets were generated.

According to Experimental Example #9, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #9 were as shown in [Table 12] and [Table 13] below.

**[Table 12]**

| | First value | | Second value | Accuracy | Sensitivity | Specificity | PPV | NPV |
|---|---|---|---|---|---|---|---|---|
| 1 | Rate of change in heart rate average | ÷ | Second reference date TSH | 82.97 | 87.84 | 82.05 | 48.15 | 97.26 |
| 2 | Amount of change in relative standard deviation | x | Amount of change in heart rate skewness | 82.54 | 85.14 | 82.05 | 47.37 | 96.68 |
| 3 | Second reference date TSH | x | Amount of change in relative standard deviation | 84.48 | 87.84 | 83.85 | 50.78 | 97.32 |
| 4 | Rate of change in heart rate average | x | JS divergence | 84.48 | 89.19 | 83.59 | 50.77 | 97.60 |
| 5 | Second reference date free T4 | ÷ | Second reference date TSH | 83.84 | 89.19 | 82.82 | 49.62 | 97.58 |
| 6 | JS divergence | ÷ | Second reference date TSH | 83.41 | 87.84 | 82.56 | 48.87 | 97.28 |
| 7 | Amount of change in heart rate kurtosis | ÷ | Amount of change in relative standard deviation | 83.41 | 89.19 | 82.31 | 48.89 | 97.57 |
| 8 | Rate of change in heart rate average | ÷ | Amount of change in heart rate skewness | 84.05 | 89.19 | 83.08 | 50.00 | 97.59 |
| 9 | Rate of change in heart rate average | ÷ | JS divergence | 84.27 | 87.84 | 83.59 | 50.39 | 97.31 |
| 10 | Second reference date free T4 | ÷ | Amount of change in heart rate kurtosis | 84.48 | 89.19 | 83.59 | 50.77 | 97.60 |
| 11 | Amount of change in relative standard deviation | ÷ | Second reference date free T4 | 84.27 | 87.84 | 83.59 | 50.39 | 97.31 |
| 12 | Second reference date TSH | ÷ | Second reference date free T4 | 84.27 | 87.84 | 83.59 | 50.39 | 97.31 |
| 13 | Amount of change in relative standard deviation | ÷ | Rate of change in heart rate average | 83.41 | 89.19 | 82.31 | 48.89 | 97.57 |
| 14 | Amount of change in heart rate skewness | ÷ | Second reference date free T4 | 83.41 | 87.84 | 82.56 | 48.87 | 97.28 |
| 15 | Second reference date TSH | ÷ | Amount of change in heart rate skewness | 83.19 | 86.49 | 82.56 | 48.48 | 96.99 |
| 16 | Second reference date TSH | ÷ | Amount of change in relative standard deviation | 84.05 | 87.84 | 83.33 | 50.00 | 97.31 |
| 17 | Rate of change in heart rate average | ÷ | Amount of change in heart rate kurtosis | 83.62 | 89.19 | 82.56 | 49.25 | 97.58 |
| 18 | Amount of change in heart rate kurtosis | x | JS divergence | 84.48 | 87.84 | 83.85 | 50.78 | 97.32 |
| 19 | Amount of change in heart rate skewness | ÷ | Second reference date TSH | 83.62 | 89.19 | 82.56 | 49.25 | 97.58 |
| 20 | Second reference date TSH | x | Amount of change in heart rate skewness | 84.27 | 90.54 | 83.08 | 50.38 | 97.89 |
| 21 | Second reference date free T4 | x | Amount of change in heart rate kurtosis | 83.84 | 87.84 | 83.08 | 49.62 | 97.30 |
| 22 | Amount of change in relative standard deviation | ÷ | JS divergence | 83.84 | 89.19 | 82.82 | 49.62 | 97.58 |
| 23 | Amount of change in relative standard deviation | x | JS divergence | 84.05 | 87.84 | 83.33 | 50.00 | 97.31 |
| 24 | Second reference date free T4 | x | Amount of change in heart rate skewness | 83.41 | 86.49 | 82.82 | 48.85 | 97.00 |
| 25 | Second reference date free T4 | ÷ | Rate of change in heart rate average | 84.27 | 89.19 | 83.33 | 50.38 | 97.60 |
| 26 | Amount of change in heart rate kurtosis | ÷ | Rate of change in heart rate average | 83.19 | 86.49 | 82.56 | 48.48 | 96.99 |
| 27 | JS divergence | ÷ | Amount of change in heart rate skewness | 83.84 | 89.19 | 82.82 | 49.62 | 97.58 |
| 28 | Amount of change in heart rate skewness | ÷ | Amount of change in relative standard deviation | 83.84 | 87.84 | 83.08 | 49.62 | 97.30 |
| 29 | Second reference date free T4 | x | JS divergence | 84.05 | 89.19 | 83.08 | 50.00 | 97.59 |
| 30 | Rate of change in heart rate average | x | Second reference date free T4 | 84.05 | 87.84 | 83.33 | 50.00 | 97.31 |
| 31 | Rate of change in heart rate average | x | Amount of change in relative standard deviation | 83.62 | 89.19 | 82.56 | 49.25 | 97.58 |
| 32 | Amount of change in heart rate skewness | x | Amount of change in heart rate kurtosis | 83.62 | 86.49 | 83.08 | 49.23 | 97.01 |
| 33 | Second reference date TSH | ÷ | JS divergence | 83.19 | 89.19 | 82.05 | 48.53 | 97.56 |
| 34 | Amount of change in relative standard deviation | ÷ | Amount of change in heart rate skewness | 83.62 | 89.19 | 82.56 | 49.25 | 97.58 |
| 35 | Second reference date free T4 | x | Second reference date TSH | 83.62 | 87.84 | 82.82 | 49.24 | 97.29 |
| 36 | Rate of change in heart rate average | ÷ | Amount of change in relative standard deviation | 84.27 | 89.19 | 83.33 | 50.38 | 97.60 |
| 37 | Amount of change in heart rate kurtosis | ÷ | Amount of change in heart rate skewness | 83.84 | 89.19 | 82.82 | 49.62 | 97.58 |
| 38 | Second reference date TSH | ÷ | Rate of change in heart rate average | 84.48 | 89.19 | 83.59 | 50.77 | 97.60 |
| 39 | JS divergence | ÷ | Rate of change in heart rate average | 84.48 | 89.19 | 83.59 | 50.77 | 97.60 |

**[Table 13]**

| | | | | | | | | |
|---|---|---|---|---|---|---|---|---|
| 40 | Second reference date TSH | x | Amount of change in heart rate kurtosis | 83.84 | 87.84 | 83.08 | 49.62 | 97.30 |
| 41 | Rate of change in heart rate average | x | Amount of change in heart rate skewness | 84.48 | 89.19 | 83.59 | 50.77 | 97.60 |
| 42 | Rate of change in heart rate average | x | Amount of change in heart rate kurtosis | 84.70 | 89.19 | 83.85 | 51.16 | 97.61 |
| 43 | JS divergence | ÷ | Amount of change in heart rate kurtosis | 84.05 | 86.49 | 83.59 | 50.00 | 97.02 |
| 44 | Second reference date free T4 | ÷ | JS divergence | 85.34 | 90.54 | 84.36 | 52.34 | 97.92 |
| 45 | Amount of change in heart rate kurtosis | ÷ | Second reference date free T4 | 83.62 | 87.84 | 82.82 | 49.24 | 97.29 |
| 46 | Amount of change in relative standard deviation | ÷ | Amount of change in heart rate kurtosis | 83.41 | 89.19 | 82.31 | 48.89 | 97.57 |
| 47 | Rate of change in heart rate average | x | Second reference date TSH | 83.62 | 87.84 | 82.82 | 49.24 | 97.29 |
| 48 | Amount of change in heart rate skewness | ÷ | JS divergence | 84.48 | 87.84 | 83.85 | 50.78 | 97.32 |
| 49 | Second reference date free T4 | x | Amount of change in relative standard deviation | 84.27 | 90.54 | 83.08 | 50.38 | 97.89 |
| 50 | Amount of change in heart rate skewness | x | JS divergence | 84.27 | 87.84 | 83.59 | 50.39 | 97.31 |
| 51 | JS divergence | ÷ | Amount of change in relative standard deviation | 84.70 | 86.49 | 84.36 | 51.20 | 97.05 |
| 52 | Amount of change in relative standard deviation | x | Amount of change in heart rate kurtosis | 83.62 | 87.84 | 82.82 | 49.24 | 97.29 |
| 53 | Amount of change in relative standard deviation | ÷ | Second reference date TSH | 83.62 | 87.84 | 82.82 | 49.24 | 97.29 |
| 54 | Second reference date free T4 | ÷ | Amount of change in heart rate skewness | 83.19 | 87.84 | 82.31 | 48.51 | 97.27 |
| 55 | JS divergence | ÷ | Second reference date free T4 | 84.05 | 87.84 | 83.33 | 50.00 | 97.31 |
| 56 | Amount of change in heart rate kurtosis | ÷ | JS divergence | 84.05 | 86.49 | 83.59 | 50.00 | 97.02 |
| 57 | Second reference date TSH | x | JS divergence | 84.05 | 87.84 | 83.33 | 50.00 | 97.31 |
| 58 | Amount of change in heart rate skewness | ÷ | Amount of change in heart rate kurtosis | 83.84 | 87.84 | 83.08 | 49.62 | 97.30 |
| 59 | Amount of change in heart rate skewness | ÷ | Rate of change in heart rate average | 84.70 | 89.19 | 83.85 | 51.16 | 97.61 |
| 60 | Second reference date TSH | ÷ | Amount of change in heart rate kurtosis | 84.05 | 86.49 | 83.59 | 50.00 | 97.02 |
| 61 | Second reference date free T4 | ÷ | Amount of change in relative standard deviation | 83.84 | 90.54 | 82.56 | 49.63 | 97.87 |
| 62 | Amount of change in heart rate kurtosis | ÷ | Second reference date TSH | 83.62 | 89.19 | 82.56 | 49.25 | 97.58 |
| 63 | Rate of change in heart rate average | ÷ | Second reference date free T4 | 84.70 | 89.19 | 83.85 | 51.16 | 97.61 |

### (11) Experimental Example #10 - Use of Personal Information of Subject

### Preparation of Training Data Sets

A total of 563 clinical data sets collected from a total of 171 patients were used.

According to the method of training the hyperthyroidism prediction model described above in Training Method Embodiment #11, the model was trained.

In particular, training was performed using training data sets such as (1) [(a period average heart rate corresponding to a first test date - a period average heart rate corresponding to a second test date), a free T4 concentration corresponding to the second test date, a subject's age, the subject's sex, a diagnosis result (hyperthyroidism or non-hyperthyroidism) corresponding to the first test date]. In addition, training was performed using training data sets such as (2) [((a period average heart rate corresponding to a first test date - a period average heart rate corresponding to a second test date) / the period average heart rate corresponding to the second test date), a free T4 concentration corresponding to the second test date, a subject's age, the subject's sex, a diagnosis result (hyperthyroidism or non-hyperthyroidism) corresponding to the first test date]. Furthermore, training was performed using training data sets such as (3) [(a period average heart rate corresponding to a first test date - a period average heart rate corresponding to a second test date), a free T4 concentration corresponding to the second test date, a TSH concentration corresponding to the second test date, a subject's age, the subject's sex, a diagnosis result (hyperthyroidism or non-hyperthyroidism) corresponding to the first test date]. In addition, training was performed using training data sets such as (4) [((a period average heart rate corresponding to a first test date - a period average heart rate corresponding to a second test date) / the period average heart rate corresponding to the second test date), a free T4 concentration corresponding to the second test date, a TSH concentration corresponding to the second test date, a subject's age, the subject's sex, a diagnosis result (hyperthyroidism or non-hyperthyroidism) corresponding to the first test date]. Lastly, training was performed using training data sets such as (5) [((a period average heart rate corresponding to a first test date - a period average heart rate corresponding to a second test date) / the period average heart rate corresponding to the second test date), (a relative standard deviation of interval heart rates corresponding to the first test date - a relative standard deviation of interval heart rates corresponding to the second test date), (the skewness of interval heart rates corresponding to the second test date - the skewness of interval heart rates corresponding to the first test date), (the kurtosis of interval heart rates corresponding to the second test date - the kurtosis of interval heart rates corresponding to the first test date), (a JS divergence between interval heart rates corresponding to the second test date and interval heart rates corresponding to the first test date), a free T4 concentration corresponding to the second test date, a TSH concentration corresponding to the second test date, a subject's age, the subject's sex, a diagnosis result (hyperthyroidism or non-hyperthyroidism) corresponding to the first test date].

Herein, regarding the period average heart rate corresponding to the test date, resting heart rates for 10 days were obtained on the basis of the test date, and an average of the obtained resting heart rates was calculated.

Herein, the training data sets were not generated using a "difference" in clinical data between different patients, but the training data sets were generated using clinical data sets obtained from one patient.

Herein, as the clinical data used for training, 563 pieces of data secured from 171 patients were used, and a total of 1,542 training data sets were generated therefrom.

### Training of Hyperthyroidism Prediction Model

Using the light gradient boosting machine, the hyperthyroidism prediction model was trained with the above-described training data sets.

### Test of Hyperthyroidism Prediction Model

The test data sets used to determine the accuracy of the trained prediction model were generated from 464 pieces of clinical data secured from a total of 126 patients, and a total of 1,416 test data sets were generated.

According to Experimental Example #10, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #10 were as shown in [Table 14] below.

**[Table 14]**

| | Accuracy | Sensitivity | Specificity | PPV | NPV |
|---|---|---|---|---|---|
| (1) | 84.27 | 53.33 | 85.30 | 10.81 | 98.21 |
| (2) | 83.41 | 53.33 | 84.41 | 10.26 | 98.19 |
| (3) | 83.41 | 53.33 | 84.41 | 10.26 | 98.19 |
| (4) | 84.48 | 53.33 | 85.52 | 10.96 | 98.21 |
| (5) | 85.99 | 60.00 | 86.86 | 13.24 | 98.48 |

### (12) Experimental Example #11 - Experiment on Length of Predetermined Period

### Preparation of Training Data Sets

A total of 563 clinical data sets collected from a total of 171 patients were used.

According to the method of training the hyperthyroidism prediction model described above in Training Method Embodiment #10, the model was trained.

In particular, an amount of change in a relative standard deviation, an amount of change in skewness, an amount of change in kurtosis, and a JS divergence were used as parameters for determining a change in distribution. Furthermore, (a free T4 concentration on a second reference date / a JS divergence) was used as a combination value of variables. That is, generated were training data sets in the format such as [((a period average heart rate corresponding to a first test date - a period average heart rate corresponding to a second test date) / the period average heart rate corresponding to the second test date), (a relative standard deviation of interval heart rates corresponding to the first test date - a relative standard deviation of interval heart rates corresponding to the second test date), (the skewness of interval heart rates corresponding to the second test date - the skewness of interval heart rates corresponding to the first test date), (the kurtosis of interval heart rates corresponding to the second test date - the kurtosis of interval heart rates corresponding to the first test date), (a JS divergence between interval heart rates corresponding to the second test date and the first test date), a day gap between the second test date and the first test date, a thyroid hormone concentration corresponding to the second test date, (a free T4 concentration on the second test date / the JS divergence between the interval heart rates corresponding to the second test date and the first test date), a diagnosis result (hyperthyroidism or non-hyperthyroidism) corresponding to the first test date], and the training data sets were used in training the model.

In Experimental Example #11, while changing a predetermined period for calculating a period average heart rate corresponding to the test date to 1, 5, 10, 15, 25, and 30 days, the effect on accuracy was determined.

In addition, regarding the used hormone level, a method using both a free T4 and a TSH concentration was used.

Herein, the training data sets were not generated using a "difference" in clinical data between different patients, but the training data sets were generated using clinical data sets obtained from one patient.

Herein, as the clinical data used for training, 563 pieces of data secured from 171 patients were used, and a total of 1,542 training data sets were generated therefrom.

### Training of Hyperthyroidism Prediction Model

Using the light gradient boosting machine, the hyperthyroidism prediction model was trained with the above-described training data sets.

### Test of Hyperthyroidism Prediction Model

The test data sets used to determine the accuracy of the trained prediction model were generated from 464 pieces of clinical data secured from a total of 126 patients, and a total of 1,416 test data sets were generated.

According to Experimental Example #11, the trained hyperthyroidism prediction model was tested for accuracy using the above-described test sets.

The accuracy, sensitivity, specificity, positive predictive value (PPV), and negative predictive value (NPV) of the hyperthyroidism prediction model according to Experimental Example #11 were as shown in [Table 15] below.

**[Table 15]**

| Predetermined period (days) | Accuracy | Sensitivity | Specificity | PPV | NPV |
|---|---|---|---|---|---|
| 1 | 80.60 | 71.62 | 82.31 | 43.44 | 93.86 |
| 5 | 81.90 | 74.32 | 83.33 | 45.83 | 94.48 |
| 10 | 83.41 | 79.73 | 84.10 | 48.76 | 95.63 |
| 15 | 86.21 | 81.08 | 87.18 | 54.55 | 96.05 |
| 20 | 84.48 | 85.14 | 84.36 | 50.81 | 96.76 |
| 25 | 82.54 | 81.08 | 82.82 | 47.24 | 95.85 |
| 30 | 82.97 | 79.73 | 83.59 | 47.97 | 95.60 |

The inventors of the present application found that in predicting hyperthyroidism, the highest accuracy is achieved when 15 days are used as a predetermined period for calculating interval heart rates.

The method of training the thyroid dysfunction prediction model, the method for predicting thyroid dysfunction by using the model, and the system therefor have been described.

Hereinafter, a method of training a model of predicting a thyroid hormone level, a method for predicting thyroid dysfunction by using the model, and a system therefor will be briefly described.

### 6. Method of Training Thyroid Hormone Prediction Model

### Training Method Embodiment #12. Difference Value from Heart Rates on Reference Date, and Hormone Value on Reference Date

According to a 12th exemplary embodiment described in the present application, in order to train a learning model, a value obtained by subtracting, from a period average heart rate corresponding to a first test date, a period average heart rate corresponding to a second test date, and a hormone on the second test date may be used as input values, and a hormone concentration corresponding to the first test date may be used as a labeling value.

Herein, as the learning model, a machine learning model capable of regression may be used. For example, as the learning model, light gradient boosting machine, support vector machine, random forest, extra trees, ada boost, extreme gradient boosting, Cat-Boost, etc. may be used.

The thyroid hormone concentration prediction model includes at least one selected from a group consisting of a TRH concentration prediction model, a TSH concentration prediction model, a T4 concentration prediction model, a free T4 concentration prediction model, a T3 concentration prediction model, and a free T3 concentration prediction model with respect to a target date.

In calculating a period average heart rate, resting heart rates for a predetermined period based on a particular date may be used, wherein the predetermined period may be one of the following: 1, 2, 3, 4, 5, 6, 7, 8, 9, 10, 11, 12, 13, 14, 15, 16, 17, 18, 19, 20, 21, 22, 23, 24, 25, 26, 27, 28, 29, and 30 days.

According to the type of the thyroid hormone concentration prediction model, a concentration value corresponding to the type among a plurality of hormone concentrations corresponding to the first test date may be used as the labeling value. For example, when it is intended to train the free T4 concentration prediction model, a free T4 concentration value corresponding to the first test date is used as the labeling value. As another example, when it is intended to train the TSH concentration prediction model, a TSH concentration value corresponding to the first test date is used as the labeling value.

Hereinafter, for convenience of description, a description will be given based on the case of training the free T4 concentration prediction model.

According to the 12th exemplary embodiment, in order to train the free T4 concentration prediction model, secured clinical data may be used to generate training data sets in the format such as [(a period average heart rate corresponding to a first test date - a period average heart rate corresponding to a second test date), a thyroid hormone concentration corresponding to the second test date, a free T4 concentration corresponding to the first test date]. Herein, the thyroid hormone concentration corresponding to the second test date may be one selected from the group of a TRH concentration, a TSH concentration, a T4 concentration, a free T4 concentration, a T3 concentration, and a free T3 concentration, or may be a combination thereof.

For example, assuming that a total of three data sets were obtained from a first patient, that for the first test, a determined free T4 hormone concentration was a first free T4 concentration and a determined TSH concentration was a first TSH concentration, that for the second test, a determined free T4 hormone concentration was a second free T4 concentration and a determined TSH concentration was a second TSH concentration, and that for the third test, a determined free T4 hormone concentration was a third free T4 concentration and a determined TSH concentration was a third TSH concentration, training data sets that may be obtained from the clinical data of the patient may be as follows.
[(a period average heart rate corresponding to a first test date - a period average heart rate corresponding to a second test date), a second free T4 concentration, a second TSH concentration, a first free T4 concentration],
[(the period average heart rate corresponding to the first test date - a period average heart rate corresponding to a third test date), a third free T4 concentration, a third TSH concentration, the first free T4 concentration],
[(the period average heart rate corresponding to the second test date - the period average heart rate corresponding to the first test date), the first free T4 concentration, a first TSH concentration, the second free T4 concentration],
[(the period average heart rate corresponding to the second test date - the period average heart rate corresponding to the third test date), the third free T4 concentration, the third TSH concentration, the third free T4 concentration],
[(the period average heart rate corresponding to the third test date - the period average heart rate corresponding to the first test date), the first free T4 concentration, the first TSH concentration, the third free T4 concentration], and
[(the period average heart rate corresponding to the third test date - the period average heart rate corresponding to the second test date), the second free T4 concentration, the second TSH concentration, the third free T4 concentration]

As another example, assuming that a total of four data sets were obtained from a second patient, that for the first test, a determined free T4 hormone concentration was a first free T4 concentration and a determined TSH concentration was a first TSH concentration, that for the second test, a determined free T4 hormone concentration was a second free T4 concentration and a determined TSH concentration was a second TSH concentration, that for the third test, a determined free T4 hormone concentration was a third free T4 concentration and a determined TSH concentration was a third TSH concentration, and that for the fourth test, a determined free T4 hormone concentration was a fourth free T4 concentration and a determined TSH concentration was a fourth TSH concentration, training data sets that may be obtained from the clinical data of the patient may be as follows.
[(a period average heart rate corresponding to a first test date - a period average heart rate corresponding to a second test date), a second free T4 concentration, a second TSH concentration, a first free T4 concentration],
[(the period average heart rate corresponding to the first test date - a period average heart rate corresponding to a third test date), a third free T4 concentration, a third TSH concentration, the first free T4 concentration],
[(the period average heart rate corresponding to the first test date - a period average heart rate corresponding to a fourth test date), a fourth free T4 concentration, a fourth TSH concentration, the first free T4 concentration],
[(the period average heart rate corresponding to the second test date - the period average heart rate corresponding to the first test date), the first free T4 concentration, a first TSH concentration, the second free T4 concentration],
[(the period average heart rate corresponding to the second test date - the period average heart rate corresponding to the third test date), the third free T4 concentration, the third TSH concentration, the second free T4 concentration],
[(the period average heart rate corresponding to the second test date - the period average heart rate corresponding to the fourth test date), the fourth free T4 concentration, the fourth TSH concentration, the second free T4 concentration],
[(the period average heart rate corresponding to the third test date - the period average heart rate corresponding to the first test date), the first free T4 concentration, the first TSH concentration, the third free T4 concentration],
[(the period average heart rate corresponding to the third test date - the period average heart rate corresponding to the second test date), the second free T4 concentration, the second TSH concentration, the third free T4 concentration],
[(the period average heart rate corresponding to the third test date - the period average heart rate corresponding to the fourth test date), the fourth free T4 concentration, the fourth TSH concentration, the third free T4 concentration],
[(the period average heart rate corresponding to the fourth test date - the period average heart rate corresponding to the first test date), the first free T4 concentration, the first TSH concentration, the fourth free T4 concentration],
[(the period average heart rate corresponding to the fourth test date - the period average heart rate corresponding to the second test date), the second free T4 concentration, the second TSH concentration, the fourth free T4 concentration], and
[(the period average heart rate corresponding to the fourth test date - the period average heart rate corresponding to the third test date), the third free T4 concentration, the third TSH concentration, the fourth free T4 concentration]

When it is intended to train a model for predicting a TSH concentration rather than a model for predicting a free T4 concentration, the input values in the above-described training data sets are maintained and a TSH concentration is used as a labeling value instead of a free T4 concentration.

### Training Method Embodiment #13. Rate of Change in Heart Rates on Reference Date, and Hormone Value on Reference Date

According to a 13th exemplary embodiment described in the present application, compared to the above-described 12th exemplary embodiment, a rate of change in a period average heart rate on a second test date with respect to a first test date is used as an input value instead of the value obtained by subtracting the period average heart rate corresponding to the second test date from the period average heart rate corresponding to the first test date, and the rest is the same as in the 12th exemplary embodiment.

### Training Method Embodiment #14. Rate of Change in Heart Rates on Reference Date, Amount of Change in Relative Standard Deviation, and Hormone Value on Reference Date

According to a 14th exemplary embodiment described in the present application, compared to the above-described 13th exemplary embodiment, a value obtained by subtracting, from a relative standard deviation of interval heart rates corresponding to a first test date, a relative standard deviation of interval heart rates corresponding to a second test date is further used as an input value, and the rest is the same as in the 13th exemplary embodiment.

### Training Method Embodiment #15. Rate of Change in Heart Rates on Reference Date, Amount of Change in Relative Standard Deviation, Amount of Change in Skewness, Amount of Change in Kurtosis, JS divergence, and Hormone Value on Reference Date

According to a 15th exemplary embodiment described in the present application, compared to the above-described 14th exemplary embodiment, a value related to a difference between a parameter related to the distribution of interval heart rates corresponding to a first test date and a parameter related to the distribution of interval heart rates corresponding to a second test date is further used as an input value, and the rest is the same as in the 14th exemplary embodiment.

### Training Method Embodiment #16. Rate of Change in Heart Rates on Reference Date, Amount of Change in Relative Standard Deviation, Amount of Change in Skewness, Amount of Change in Kurtosis, JS divergence, and Hormone Value + Day Gap on Reference Date

According to a 16th exemplary embodiment described in the present application, compared to the above-described 15th exemplary embodiment, a value related to a day gap between a first test date and a second test date is further used as an input value, and the rest is the same as in the 15th exemplary embodiment.

### 7. Method for Predicting Thyroid Dysfunction on Basis of Thyroid Hormone Prediction Model

The method for predicting thyroid dysfunction, which is described in the present application, may be performed by the above-described system 1.

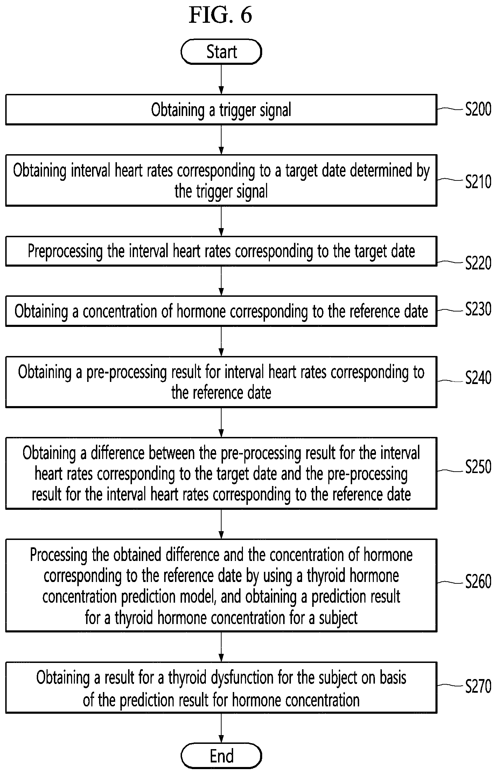

FIG. 6 is a flowchart illustrating a method for predicting thyroid dysfunction described in the present application.

Referring to FIG. 6, the method, which is described in the present application, for predicting thyroid dysfunction for a subject includes: obtaining a trigger signal in step S200; obtaining interval heart rates corresponding to a target date determined by the trigger signal, in step S210; preprocessing the interval heart rates corresponding to the target date in step S220; obtaining a hormone concentration corresponding to a reference date in step S230; obtaining a pre-processing result for interval heart rates corresponding to the reference date in step S240; obtaining a difference between the pre-processing result for the interval heart rates corresponding to the target date and the pre-processing result for the interval heart rates corresponding to the reference date in step S250; processing the obtained difference and the hormone concentration corresponding to the reference date by using a thyroid hormone concentration prediction model, and obtaining a prediction result for a thyroid hormone concentration for the subject in step S260; and obtaining a result for thyroid dysfunction for the subject on the basis of the prediction result for the hormone concentration in step S270.

Herein, the steps including obtaining a trigger signal in step S200, obtaining interval heart rates corresponding to a target date determined by the trigger signal in step S210, preprocessing the interval heart rates corresponding to the target date in step S220, obtaining a hormone concentration corresponding to a reference date in step S230, obtaining a pre-processing result for interval heart rates corresponding to the reference date in step S240, and obtaining a difference between a pre-processing result for the interval heart rates corresponding to the target date and the pre-processing result for the interval heart rates corresponding to the reference date in step S250 are respectively the same as or very similar to the steps described with reference to FIG. 5 including obtaining a trigger signal in step S100, obtaining interval heart rates corresponding to a target date determined by the trigger signal in step S110, preprocessing the interval heart rates corresponding to the target date in step S120, obtaining a hormone concentration corresponding to a reference date in step S130, obtaining a pre-processing result for interval heart rates corresponding to the reference date in step S140, and obtaining a difference between a pre-processing result for the interval heart rates corresponding to the target date and the pre-processing result for the interval heart rates corresponding to the reference date in step S150. Therefore, a detailed description will be omitted.

### The obtaining of the prediction result for the thyroid hormone concentration through the thyroid hormone concentration prediction model in step S260

### Acquisition of Prediction Result for First Thyroid Hormone Concentration through First Thyroid Hormone Concentration Prediction Model

A first thyroid hormone concentration for the subject may be obtained through a first thyroid hormone concentration prediction model. For example, the first thyroid hormone may be TRH.

### Acquisition of Prediction Result for Second Thyroid Hormone Concentration through Second Thyroid Hormone Concentration Prediction Model

A second thyroid hormone concentration for the subject may be obtained through a second thyroid hormone concentration prediction model. For example, the second thyroid hormone may be TSH.

### Acquisition of Prediction Result for Third Thyroid Hormone Concentration through Third Thyroid Hormone Concentration Prediction Model

A third thyroid hormone concentration for the subject may be obtained through a third thyroid hormone concentration prediction model. For example, the third thyroid hormone may be T4.

### Acquisition of Prediction Result for Fourth Thyroid Hormone Concentration through Fourth Thyroid Hormone Concentration Prediction Model

A fourth thyroid hormone concentration for the subject may be obtained through a fourth thyroid hormone concentration prediction model. For example, the fourth thyroid hormone may be free T4.

### Acquisition of Prediction Result for Fifth Thyroid Hormone Concentration through Fifth Thyroid Hormone Concentration Prediction Model

A fifth thyroid hormone concentration for the subject may be obtained through a fifth thyroid hormone concentration prediction model. For example, the fifth thyroid hormone may be T3.

### Acquisition of Prediction Result for Sixth Thyroid Hormone Concentration through Sixth Thyroid Hormone Concentration Prediction Model

A sixth thyroid hormone concentration for the subject may be obtained through a sixth thyroid hormone concentration prediction model. For example, the sixth thyroid hormone may be free T3.

As described above, one selected from the group of the first thyroid hormone concentration to the sixth thyroid hormone concentration, or a combination thereof may be obtained.

### The obtaining of the result for thyroid dysfunction for the subject on the basis of the prediction result for the hormone concentration in step S270

On the basis of the obtained prediction values for the hormone concentration, it may be determined whether the subject is in a hyperthyroidism state, a hypothyroidism state, or a normal state.

Ranges corresponding to 'normal', 'hyperthyroidism', and 'hypothyroidism' are predetermined for each thyroid hormone concentration, so the predetermined ranges may be used.

The heart rate measuring device 10, the user terminal 20, and the server 30 may, in an appropriately distributed manner, perform the above-described steps including: obtaining a trigger signal in step S200; obtaining interval heart rates corresponding to a target date determined by the trigger signal, in step S210; preprocessing the interval heart rates corresponding to the target date in step S220; obtaining a hormone concentration corresponding to a reference date in step S230; obtaining a pre-processing result for interval heart rates corresponding to the reference date in step S240; obtaining a difference between a pre-processing result for the interval heart rates corresponding to the target date and the pre-processing result for the interval heart rates corresponding to the reference date in step S250; processing the obtained difference and the hormone concentration corresponding to the reference date by using a thyroid hormone concentration prediction model, and obtaining a prediction result for a thyroid hormone concentration for the subject in step S260; and obtaining a result for thyroid dysfunction for the subject on the basis of the prediction result for the hormone concentration in step S270.

### 8. Experimental Examples

### Experimental Example #12.

### Preparation of Training Data Sets

A total of 563 clinical data sets collected from a total of 171 patients were used.

According to the method of training the thyroid hormone concentration prediction model described above in Training Method Embodiment #13, the model was trained. In particular, a prediction model for predicting a free T4 concentration was trained.

In the meantime, a free T4 concentration and a TSH concentration were used as hormone concentrations corresponding to each test date, and regarding a period average heart rate corresponding to the test date, resting heart rates for 10 days were obtained on the basis of the test date, and an average of the obtained resting heart rates was calculated.

Herein, the training data sets were not generated using a "difference" in clinical data between different patients, but the training data sets were generated using clinical data sets obtained from one patient.

Herein, as the clinical data used for training, 563 pieces of data secured from 171 patients were used, and a total of 1,542 training data sets were generated therefrom.

### Training of Hyperthyroidism Prediction Model

Using the extra trees, a free T4 concentration prediction model was trained with the above-described training data sets.

### Test of Hyperthyroidism Prediction Model

The test data sets used to determine the accuracy of the trained prediction model were generated from 464 pieces of clinical data secured from a total of 126 patients, and a total of 1,416 test data sets were generated.

According to Experimental Example #13, the trained free T4 concentration prediction model was tested for accuracy using the above-described test sets.

The mean squared error (MSE), mean absolute error (MAE), and mean absolute percentage error (MAPE) of the free T4 concentration prediction model according to Experimental Example #13 were as shown in [Table 16] below.

**[Table 16]**

| | MSE | MAE | MAPE |
|---|---|---|---|
| Free T4 | 0.2272 | 0.1281 | 0.1624 |

### Experimental Example #13.

The training data sets, the test data sets, and the generation method thereof used in Experimental Example #13 are the same as those described in Experimental Example #12.

However, unlike Experimental Example #12, in Experimental Example #13, the prediction model was trained according to Training Method Embodiment #14 instead of training the prediction model according to Training Method Embodiment #13.

### Test of Hyperthyroidism Prediction Model

According to Experimental Example #13, the trained free T4 concentration prediction model was tested for accuracy using the above-described test sets.

The mean squared error (MSE), mean absolute error (MAE), and mean absolute percentage error (MAPE) of the free T4 concentration prediction model according to Experimental Example #13 were as shown in [Table 17] below.

**[Table 17]**

| | MSE | MAE | MAPE |
|---|---|---|---|
| Free T4 | 0.2219 | 0.1203 | 0.1626 |

### Experimental Example #14.

The training data sets, the test data sets, and the generation method thereof used in Experimental Example #14 are the same as those described in Experimental Example #12.

However, unlike Experimental Example #12, in Experimental Example #14, the prediction model was trained according to Training Method Embodiment #15 instead of training the prediction model according to Training Method Embodiment #13.

### Test of Hyperthyroidism Prediction Model

According to Experimental Example #14, the trained free T4 concentration prediction model was tested for accuracy using the above-described test sets.

The mean squared error (MSE), mean absolute error (MAE), and mean absolute percentage error (MAPE) of the free T4 concentration prediction model according to Experimental Example #14 were as shown in [Table 18] below.

**[Table 18]**

| | MSE | MAE | MAPE |
|---|---|---|---|
| Free T4 | 0.2177 | 0.1104 | 0.1596 |

### Experimental Example #15.

The training data sets, the test data sets, and the generation method thereof used in Experimental Example #15 are the same as those described in Experimental Example #12.

However, unlike Experimental Example #12, in Experimental Example #15, the prediction model was trained according to Training Method Embodiment #16 instead of training the prediction model according to Training Method Embodiment #13.

### Test of Hyperthyroidism Prediction Model

According to Experimental Example #15, the trained free T4 concentration prediction model was tested for accuracy using the above-described test sets.

The mean squared error (MSE), mean absolute error (MAE), and mean absolute percentage error (MAPE) of the free T4 concentration prediction model according to Experimental Example #15 were as shown in [Table 19] below.

**[Table 19]**

| | MSE | MAE | MAPE |
|---|---|---|---|
| Free T4 | 0.2159 | 0.1079 | 0.1584 |

- 1:: System
- 10:: Heart rate measuring device
- 20:: User terminal
- 30:: Server

## Claims

1. A method for predicting thyroid dysfunction for a subject, comprising:
obtaining a trigger signal;
determining a target date based on the obtained trigger signal;
obtaining interval heart rates corresponding to the determined target date;
obtaining, for the subject, a first pre-processing result for the obtained interval heart rates corresponding to the determined target date, wherein the first pre-processing result includes at least one parameter related to an average and distribution of the interval heart rates corresponding to the target date;
obtaining, for the subject, at least one of concentration of hormone related to a thyroid corresponding to a reference date;
obtaining, for the subject, a second pre-processing result for interval heart rates corresponding to the reference date, wherein the second pre-processing result includes at least one parameter related to an average and distribution of the interval heart rates corresponding to the reference date;
obtaining a difference of the first pre-processing result with respect to the second pre-processing result; and
obtaining a prediction result for thyroid dysfunction obtained based on values including the at least one of concentration of hormone related to a thyroid corresponding to a reference date and the difference;
wherein the prediction result for thyroid dysfunction is a result of processing input values by a thyroid dysfunction prediction model, wherein the input values include the difference and the at least one of concentration of hormone related to a thyroid corresponding to the reference date.

2. The method of claim 1, wherein the at least one parameter related to the distribution of the interval heart rates corresponding to the target date includes standard deviation, skewness and kurtosis of the interval heart rates corresponding to the target date,
wherein the at least one parameter related to the distribution of the interval heart rates corresponding to the reference date includes standard deviation, skewness and kurtosis of the interval heart rates corresponding to the reference date.

3. The method of claim 1, wherein the difference of the first pre-processing result with respect to the second pre-processing result is one selected from a group consisting of 1) an amount of change in the average of the interval heart rates of the target date with respect to the average of the interval heart rates of the reference date, 2) a rate of change in the average of the interval heart rates of the target date with respect to the average of the interval heart rates of the reference date, 3) an amount of change in a standard deviation of the interval heart rates of the target date with respect to a standard deviation of the interval heart rates of the reference date, 4) an amount of change in a relative standard deviation of the interval heart rates of the target date with respect to a relative standard deviation of the interval heart rates of the reference date, 5) an amount of change in a skewness of the interval heart rates of the target date with respect to a skewness of the interval heart rates of the reference date, 6) an amount of change in a kurtosis of the interval heart rates of the target date with respect to a kurtosis of the interval heart rates of the reference date, 7) a JS Divergence between the interval heart rates corresponding to the reference date and the interval heart rates corresponding to the target date or a combination thereof.

4. The method of claim 1, wherein the at least one of concentration of hormone corresponding to the reference date is one selected from a group consisting of 1) concentration of thyroid stimulating hormone(TSH), 2) concentration of tetraiodothyronine(T4), 3) concentration of free T4(free T4) in serum, 4) concentration of triiodothyronine(T3), 5) concentration of free T3(free T3) in serum, 6) concentration of thyrotropin-releasing hormone(TRH) or a combination thereof.

5. The method of claim 1, wherein the method further comprises :
obtaining a first value and a second value from a group consisting of 1) an amount of change in the average of the interval heart rates of the target date with respect to the average of the interval heart rates of the reference date, 2) a rate of change in the average of the interval heart rates of the target date with respect to the average of the interval heart rates of the reference date, 3) an amount of change in a standard deviation of the interval heart rates of the target date with respect to a standard deviation of the interval heart rates of the reference date, 4) an amount of change in a relative standard deviation of the interval heart rates of the target date with respect to a relative standard deviation of the interval heart rates of the reference date, 5) an amount of change in a skewness of the interval heart rates of the target date with respect to a skewness of the interval heart rates of the reference date, 6) an amount of change in a kurtosis of the interval heart rates of the target date with respect to a kurtosis of the interval heart rates of the reference date, 7) a JS Divergence between the interval heart rates corresponding to the reference date and the interval heart rates corresponding to the target date 8) concentration of thyroid stimulating hormone(TSH) of the subject corresponding to the reference date, 9) concentration of tetraiodothyronine(T4) of the subject corresponding to the reference date, 10) concentration of free T4(free T4) in the subject's serum corresponding to the reference date, 11) concentration of triiodothyronine(T3) of the subject corresponding to the reference date, 12) concentration of free T3(free T3) in the subject's serum corresponding to the reference date, 6) concentration of thyrotropin-releasing hormone(TRH) of the subject corresponding to the reference date,
obtaining any one value among i) a value obtained by multiplying the first value by the second value, ii) a value obtained by dividing the first value by the second value and iii) a value obtained by dividing the second value by the first value.

6. The method of claim 5, wherein the obtaining the prediction result for thyroid dysfunction based on values including the at least one of concentration of hormone corresponding to the reference date and the difference is obtaining the prediction result for thyroid dysfunction based on values including at least one of concentration of hormone corresponding to the reference date, the difference and the obtained any one value,
wherein the prediction result for thyroid dysfunction is a result of processing input values by the thyroid dysfunction prediction model, wherein the input values include the difference, the obtained any one value and at least one of concentration of hormone corresponding to the reference date.

7. The method of claim 1, wherein the method further comprises :
obtaining a day gap between the target date and the reference date.

8. The method of claim 7, wherein the obtaining the prediction result for thyroid dysfunction based on values including the at least one of concentration of hormone corresponding to the reference date and the difference is obtaining the prediction result for thyroid dysfunction based on values including at least one of concentration of hormone corresponding to the reference date, the difference and the day gap between the target date and the reference date,
wherein the prediction result for thyroid dysfunction is a result of processing input values by the thyroid dysfunction prediction model, wherein the input values include the difference, the day gap between the target date and the reference date and at least one of concentration of hormone corresponding to the reference date.

9. The method of claim 1, wherein the interval heart rates corresponding to the target date are all resting heart rates corresponding to a predetermined period based on the target date,
wherein the interval heart rates corresponding to the reference date are all resting heart rates corresponding to the predetermined period based on the reference date.

10. The method of claim 9,
wherein the predetermined period is any one selected from 8,9,10,11,12,13,14,15,16 and 17 days.

11. The method of in claim 1,
wherein the thyroid dysfunction prediction model includes a hyperthyroidism prediction model and a hypothyroidism prediction model.

12. The method of claim 11, wherein the prediction result for thyroid dysfunction is determined by considering a first prediction result in which the input values including at least one hormone concentration and the difference are processed by the hyperthyroidism prediction model and a second prediction result in which the input values including at least one hormone concentration and the difference are processed by the hypothyroidism prediction model.
